# EUROPEAN PATENT SPECIFICATION

(11) **EP 3 822 628 B1**
(45) Date of publication and mention of the grant of the patent: **28.01.2026**
(21) Application number: 19833175.3
(22) Date of filing: 05.07.2019
(51) Int. Cl.: C12Q 1/00, G01N 27/327, G01N 33/487

(54) **DIAGNOSING DEVICE, ANALYSIS METHOD, AND PROGRAM**
DIAGNOSEVORRICHTUNG, ANALYSEVERFAHREN, UND PROGRAMM
DISPOSITIF DE DIAGNOSTIC, PROCÉDÉ D'ANALYSE, ET PROGRAMME

(30) Priority: 09.07.2018 JP 2018130027; 26.12.2018 JP 2018242680
(43) Date of publication of application: 19.05.2021
(73) Proprietor: National Institute for Materials Science, Tsukuba-shi, Ibaraki 305-0047 (JP)
(72) Inventor: OKAMOTO, Akihiro, Tsukuba-shi, Ibaraki 305-0047 (JP); NARADASU, Divya, Tsukuba-shi, Ibaraki 305-0047 (JP)
(74) Representative: Markfort, Iris-Anne Lucie
(86) International application number: PCT/JP2019/026797
(87) International publication number: WO 2020/013093

(56) References cited:
- EP-A1- 2 754 709
- WO-A1-02/097418
- WO-A1-2017/115444
- WO-A1-2018/034745
- WO-A1-2018/117448
- WO-A1-2018/117448
- WO-A2-2007/070093
- GB-A- 2 405 203
- JP-A- 2000 074 914
- JP-A- 2000 074 914
- JP-A- 2003 329 673
- JP-A- 2007 511 740
- JP-A- 2016 145 806
- JP-A- S57 500 899
- US-A1- 2016 202 206
- US-A1- 2018 136 228
- DIVYA NARADASU: "P1-42 Cariogenicity of Streptococcus mutans UA159 in dental cavity is promoted by biofilm acidification via extracellular electron transfeR", JOURNAL OF ORAL BIOSCIENCES, 8 August 2016 (2016-08-08), The 58th Annual Meeting of the Japanese Society of Basic Dental Medicine Program, pages 312, XP055674960, ISSN: 2187-9109
- NARADASU DIVYA ET AL: "Isolation and Characterization of Human Gut Bacteria Capable of Extracellular Electron Transport by Electrochemical Techniques", FRONTIERS IN MICROBIOLOGY, vol. 9, 15 January 2019 (2019-01-15), Lausanne, XP093325222, ISSN: 1664-302X, DOI: 10.3389/fmicb.2018.03267
- DIVYA NARADASU: "Cariogenicity of Streptococcus mutans UA 159 in dental cavity is promoted by biofilm acidification via extracellular electron transfer", JOURNAL OF ORAL BIOSCIENCES, 8 August 2016 (2016-08-08), The 58th Annual Meeting of the Japanese Society of Basic Dental Medicine Program, pages 312, XP055674960, ISSN: 2187-9109

## Description

### Technical Field

The present invention relates to a diagnosing apparatus, an analysis method, and a program.

### Background Art

Methods for diagnosing a type of an illness that has developed in a subject and/or a state of progress of the illness from body fluid or the like of the subject have been developed.

For example, methods for providing information for diagnosing, using saliva or the like obtained from the oral cavity of a subject, a state of progress of caries and a periodontal disease in the oral cavity are known.

As the technology as described above, JP 2012 024085 A describes "a method for performing semi-quantitative measurement of cariogenic bacteria in a plaque sample or a saliva sample, executed by (a) concentrating microbes contained in the plaque sample or the saliva sample as necessary; (b) causing the microbes to come into contact with a carbon source that is fermented to an acid by the cariogenic bacteria; (c) incubating the microbes and the carbon source under conditions that promote selective acid formation by the cariogenic bacteria; and (d) deciding a pH at least once within a period of 12 hours after addition of the carbon source; in which the semi-quantitative measurement of the cariogenic bacteria in the sample is executed by comparing the pH decided in the step (d) with at least one reference value".

JP 2012 024085 A discloses a known solution from the prior art. However, the present inventors have found that in the method described in JP 2012 024085 A, accurate information for determining a type of an illness that has developed in a subject and a state of progress of the illness cannot be provided in some cases. Further, the present inventors have found a problem that the method described in JP 2012 024085 A is complicated to operate and time-consuming to provide information.

Moreover, WO 2007/070093 A2 relates to a system and method that co-locates in a small flexible, configurable system and multi-level substrate sampling, rapid analysis, bio-sample storage and delivery functions to be performed on living tissues or matter obtained from living organisms. The types of the sampling may include chemical, biochemical, biological, thermal, mechanical, electrical, magnetic and optical sampling. In general, the analysis performed at the point of sampling measures the sample taken and records its value. The bio-sample storage function encapsulates a small sample of analyte and preserves it for subsequent examination or analysis, either on the organism by the system or at a remote location by an independent analysis system. Once stored, the sample can provide a record of a biological state at the precise time of sampling. The delivery at the point of sampling can include chemical, biochemical, biological, thermal, mechanical, electrical, magnetic and optical stimuli.

Furthermore, JP 2000 074914 A discloses a sugar concentration measuring apparatus by which diabetes and a sugar-tolerance-function abnormality can be detected in a noninvasive manner. The apparatus is provided with a saliva sugar sensor, a CPU device by which a measured current by the saliva sugar sensor is converted into a saliva sugar value, a read-only memory device which outputs converted data to the CPU device, a memory device, and a display device which displays a judged result. By selecting a measuring item in a fasting state and that after a postprandial state, and measuring an electrolytic current due to the reaction of glucose with an enzyme by the saliva sugar sensor, the value of saliva sugar can be calculated by the CPU device, and by comparing its calculated value with a normal value, the state of diabetes can be judged.

Finally, GB 2 405 203 A refers to systems and methods comprising a sensor for sensing a biological target and generating a signal and a simulator for using the signal and a model of the target to generate a therapeutic or diagnostic output. The sensor may be reconfigurable by the simulator. The system may also comprise a further sensor which senses food consumed by the biological target to generate a second signal that is used by the simulator, preferably in association with a regulatory condition for example an FDA guideline. Also disclosed are systems with biosensors for DNA or RNA, peptides or proteins, antibody or antigen, vector, virus, lipid, fatty acid, inorganic or electrochemical substrates. The simulation models may be for genomics, proteomics, computational chemistry, pharmacogenomics, computational biology, computational biophysics, cell behaviour, pharmacokinetics, metabolomics or transcriptomics. The system may have a neural network, fuzzy logic or similar means of programming.

In this regard, it is an object of the present technology to provide a diagnosing apparatus capable of providing, more accurately and simply, information for determining at least one item selected from the group consisting of a type of an illness that has developed in a subject, and a state of progress of the illness that has developed in the subject. Further, another object of the present technology is to provide an analysis method and a program.

### Solution to Problem

The present inventors have intensively studied to achieve the above-mentioned object, and as a result, have found that the above-mentioned object can be achieved by a diagnosing apparatus with the features of claim 1, an analysis method with the features of claim 11 or a program with the features of claim 14.

Further possible embodiments are disclosed in the description of the figures and the dependent claims.

### Advantageous Effects of Invention

In accordance with the present invention, it is possible to provide a diagnosing apparatus capable of providing, more accurately and simply, information for determining at least one item selected from the group consisting of a type of an illness that has developed in a subject, and a state of progress of the illness that has developed in the subject. Further, in accordance with the present invention, it is possible to provide an analysis method and a program.

### Brief Description of Drawings

[Fig. 1] Fig. 1 is a schematic diagram showing a diagnosing apparatus according to a first embodiment of the present invention falling under the claimed invention.
[Fig. 2] Fig. 2 is a transmission electron microscopy image of a section stained under acidic conditions (pH4), i.e., to be redox reaction-specific to cultured Streptococcus mutans bacteria.
[Fig. 3] Fig. 3 is a transmission electron microscopy image of a section stained to be redox reaction-specific to Streptococcus mutans bacteria cultured under neutral conditions.
[Fig. 4] Fig. 4 is a diagram showing a temporal change in the glucose oxidation current in the case where a solution containing bacteria and glucose is measured using a diagnosing apparatus according to an embodiment of the present invention.
[Fig. 5] Fig. 5 is an electron micrograph of Capnocytophaga ochracea bacteria cultured under anaerobic conditions.
[Fig. 6] Fig. 6 is a diagram showing a temporal change in the current of Capnocytophaga ochracea bacteria cultured under anaerobic conditions.
[Fig. 7] Fig. 7 is an explanation diagram for describing an example of a method of performing comparison by an analysis output unit.
[Fig. 8] Fig. 8 is a schematic graph showing the magnitude of the current value with respect to the potential difference between electrodes of a sample solution D3 and a sample solution D4 in the case where a sweep voltage is applied between the electrodes.
[Fig. 9] Fig. 9 shows an example of an analytical flow using the diagnosing apparatus according to the embodiment of the present invention.
[Fig. 10] Fig. 10 shows an example of another analytical flow using the diagnosing apparatus according to the embodiment of the present invention.
[Fig. 11] Fig. 11 is a perspective view showing a biosensor of a diagnosing apparatus according to a second embodiment of the present invention.
[Fig. 12] Fig. 12 is a perspective view showing a state in which an upper insulating substrate of the biosensor of Fig. 11 is separated.
[Fig. 13] Fig. 13 is a top view of the diagnosing apparatus according to the second embodiment of the present invention falling under the claimed invention.
[Fig. 14] Fig. 14 is a diagram showing an example of display content in the case where the current value in the sample solution is greater than or equal to a reference.
[Fig. 15] Fig. 15 is a diagram showing another example of display content in the case where the current value in the sample solution is greater than or equal to the reference.
[Fig. 16] Fig. 16 is a diagram showing yet another example of display content in the case where the current value in the sample solution is greater than or equal to the reference.
[Fig. 17] Fig. 17 is a diagram describing a functional configuration of a diagnosing apparatus including a measuring device and a biosensor.
[Fig. 18] Fig. 18 is a top view showing another embodiment of a biosensor that can be used in the diagnosing apparatus according to the embodiment of the present invention.
[Fig. 19] Fig. 19 is a top view schematically showing a state in which the upper insulating substrate of the biosensor of Fig. 18 has been removed.
[Fig. 20] Fig. 20 is a schematic diagram showing an embodiment of a comb-shaped electrode.
[Fig. 21] Fig. 21 is a schematic diagram showing another embodiment of the comb-shaped electrode.
[Fig. 22] Fig. 22 is a schematic diagram showing a diagnosing apparatus according to a third embodiment of the present invention falling under the claimed invention.
[Fig. 23] Fig. 23 is a functional explanatory diagram of a diagnosing apparatus according to a fourth embodiment of the present invention falling under the claimed invention, including a measuring device, a biosensor, and a reference creating device configured to create a reference by machine-learning on the basis of a current value.
[Fig. 24] Fig. 24 is a schematic diagram showing a system including a diagnosing apparatus, the diagnosing apparatus according to a fifth embodiment of the present invention falling under the claimed invention.
[Fig. 25] Fig. 25 is a schematic diagram of a system that includes a diagnosing apparatus and a server that is a computer, the diagnosing apparatus and the server being configured to be capable of communicating with each other via a network.
[Fig. 26] Fig. 26 is a process flow of a program according to a first embodiment thereof, the program according to an embodiment of the present invention falling under the claimed invention.
[Fig. 27] Fig. 27 shows an example of displaying that microbiota analysis is necessary, which is displayed on a diagnosing apparatus.
[Fig. 28] Fig. 28 is a schematic diagram showing a system that includes a diagnosing apparatus, a server that is a computer, and an analysis device capable of performing microbiota analysis, the diagnosing apparatus, the server, and the analysis device being configured to be capable of communicating with each other via a network.
[Fig. 29] Fig. 29 is a process flow of a program according to a second embodiment thereof, the program according to another embodiment of the present invention falling under the claimed invention.

### Mode(s) for Carrying Out the Invention

Hereinafter, the present invention will be described in detail.

Description of the components set forth below is made on the basis of typical embodiments of the present invention in some cases, but the present invention is not limited to such embodiments.

Note that in the present specification, a numerical value range represented by "to" means a range including the numerical values described before and after "to" as the lower limit value and the upper limit value.

Further, in the present specification, a state of progress of the illness means at least one item selected from the group consisting of presence or absence of the illness, the risk of the illness (risk of morbidity), and degree of progress of the illness.

### [First embodiment of diagnosing apparatus]

A diagnosing apparatus according to a first embodiment of the present invention falling under the claimed invention is a diagnosing apparatus including: a cell configured to introduce a sample solution containing body fluid collected from a subject; at least two electrodes that are disposed in the cell and come into contact with the sample solution; a voltage applying unit configured to apply a voltage between the electrodes; a measuring unit configured to measure an electrochemical response when the voltage is applied; a storage unit configured to store a reference that defines a relationship between a numerical value obtained from the electrochemical response and at least one item selected from the group consisting of a type of an illness and a state of progress of the illness; and an analysis output unit configured to compare the numerical value obtained from the subject with the reference, and provide information for determining at least one item selected from the group consisting of the type of the illness that has developed in the subject, and the state of progress of the illness that has developed in the subject.

In the following, first, as one embodiment of the present invention, cases where the body fluid is saliva and the illness is caries and a periodontal disease will be described.

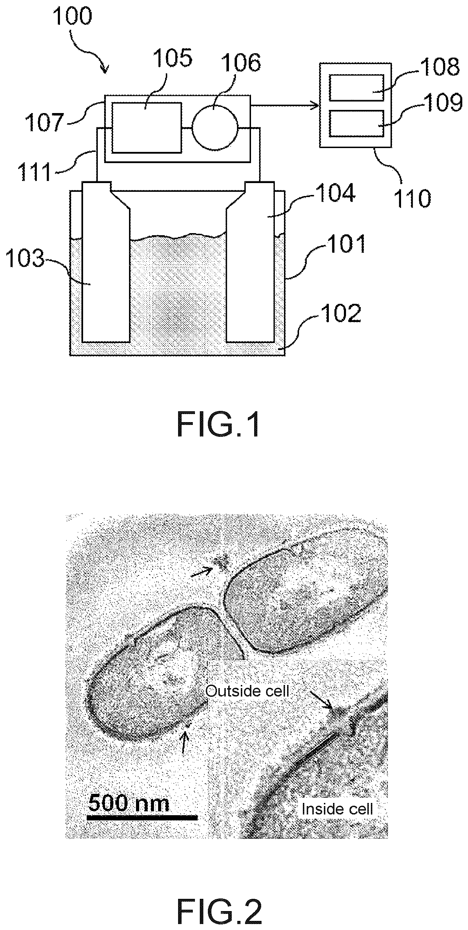

Fig. 1 is a schematic diagram showing a diagnosing apparatus according to the first embodiment of the present invention falling under the claimed invention. A diagnosing apparatus 100 includes a cell 101 for introducing a sample solution 102 containing saliva obtained from the oral cavity of a subject, and a first electrode 103 and a second electrode 104 are disposed in the cell 101 so as to come into contact with the introduced sample solution 102.

The first electrode 103 and the second electrode 104 are electrically connected to each other via a circuit 111, and a voltage applying unit 105 for applying a voltage between the first electrode 103 and the second electrode 104 is disposed on the circuit 111. Further, a measuring unit 106 for measuring an electrical response when a voltage is applied between the first electrode 103 and the second electrode 104 by the voltage applying unit 105, which is typically a current value flowing between the electrodes (the circuit 111), is disposed in the same manner.

In the diagnosing apparatus 100, the voltage applying unit 105 and the measuring unit 106 constitute a potentiostat 107.

Note that although the voltage applying unit 105 and the measuring unit 106 in the diagnosing apparatus 100 constitute the potentiostat 107, the diagnosing apparatus according to the embodiment of the present invention is not limited to the above, and the voltage applying unit 105 and the measuring unit 106 may be independently provided.

The diagnosing apparatus 100 includes a terminal 110 connected to the potentiostat 107 so as to be capable of performing data communication with each other, and the terminal 110 includes a storage unit 108 and an analysis output unit 109.

Note that the terminal 110 further includes a control unit (not shown) that is a processor, and the control unit controls the storage unit 108 and the analysis output unit 109. Further, the control unit may control the potentiostat 107.

Note that the terminal 110 is typically a computer including a display device (display unit). As the processor of the control unit, a processor such as a CPU (central processing unit) and a GPU (graphics processing unit) can be used. The control unit reads the program stored in the storage unit and performs predetermined arithmetic processing in accordance with the program.

Further, the control unit is capable of writing the operation result obtained by the analysis output unit to the storage unit as appropriate and reading it from the storage unit.

Further, the storage function of the storage unit can be realized by a non-volatile memory such as an HDD (hard disk drive) and an SSD (solid state drive). Further, the storage unit may have a function as a memory for writing or reading intermediate progress or the like of the arithmetic processing by the control unit. The memory function of the storage unit can be realized by a volatile memory such as a RAM (Random Access memory) and a DRAM (Dynamic Random Access memory).

Using the above-mentioned diagnosing apparatus 100, the procedures for providing information for determining a state of progress of an illness in the oral cavity of a subject and the principles of measurement are described below.

In the past, the acidification of biofilms responsible for caries has been thought to be caused by lactic acid generated by bacteria by degrading sugars. However, the present inventors have found for the first time that the acidification of the biofilm progresses as electric bacteria move electrons to electrodes.

Further, the present inventors have found that Streptococcus mutans and Streptococcus sobrinus, which are typical caries-causing bacteria, and the like express electrochemical activity specifically under acidic conditions and perform current generation.

The present invention is completed on the basis of the new finding that "pathogenic" caries-causing bacteria can be specifically and electrochemically detected.

Fig. 2 shows a transmission electron microscopy image of a section stained under acidic conditions (pH4), i.e., to be redox reaction-specific to cultured Streptococcus mutans bacteria. The surface of the cell wall and the inner cell membrane are stained, suggesting that an enzyme having electron transfer ability is expressed.

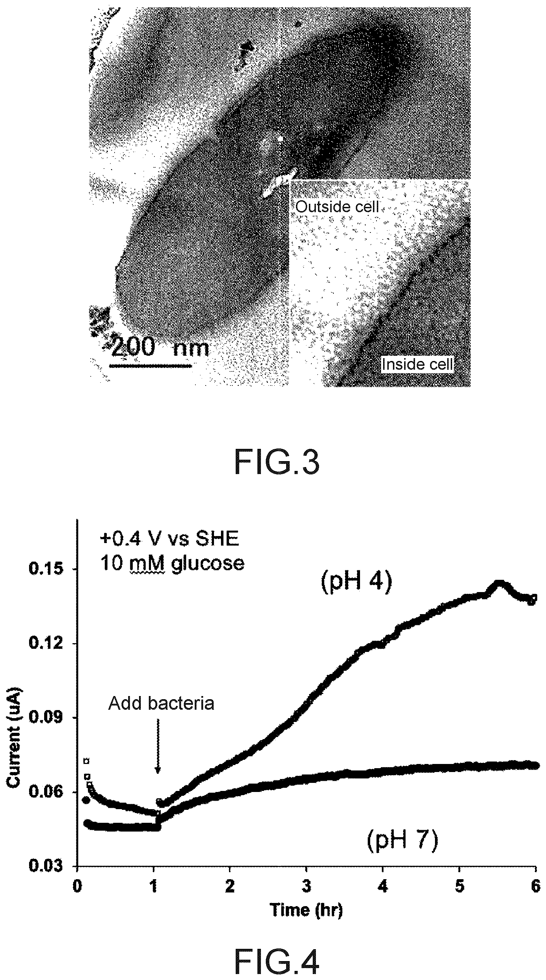

Fig. 3 shows a transmission electron microscopy image of a section stained to be redox reaction-specific to Streptococcus mutans bacteria cultured under neutral conditions. Although the cell membrane inside the cell wall is stained, the contrast with the inside of the cell is weak as compared with Fig. 2, and the surface of the cell wall is not stained, which suggests that the enzyme having the electron transfer ability is not expressed under the neutral culture conditions as compared with the acidic culture conditions.

Next, Fig. 4 shows a temporal change (i.e., electrochemical response when a voltage is applied) in the glucose oxidation current in the case where a solution containing the bacteria and glucose is measured using the diagnosing apparatus according to the embodiment of the present invention. Note that an electrode formed of indium tin oxide was used for the electrode.

In accordance with Fig. 4, it was shown that the current value was raised specifically (line described as "pH4" in Fig. 4) only under acidic conditions, i.e., when the bacteria are in the state that exerts the pathogenicity (hereinafter, the microbe in such a state will be referred to also as "pathogenic microbes" in the present specification). This is presumably because as shown in Fig. 2 and Fig. 3, electron-transfer enzymes are expressed specifically in the extracellular membrane of Streptococcus mutans bacteria under acidic conditions.

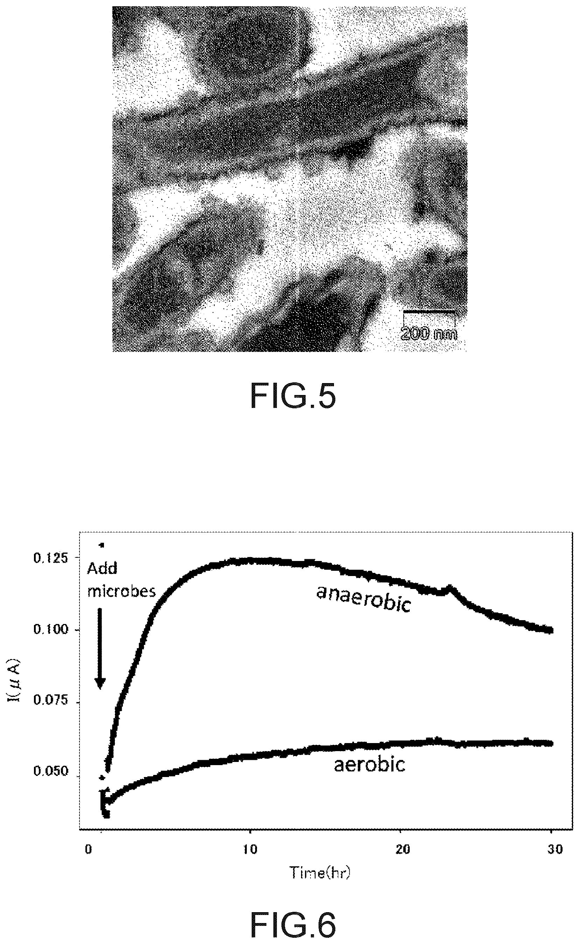

The present inventors have confirmed that this analysis method can be applied to microbes responsible for an illness in the oral cavity other than caries-causing bacteria. Fig. 5 is an electron micrograph of Capnocytophaga ochracea bacteria cultured under anaerobic conditions. Although Capnocytophaga ochracea bacteria are each a type of so-called opportunistic bacterium, it is known to be responsible for a periodontal disease.

The expression of electron-transfer enzymes has been confirmed by culturing Capnocytophaga ochracea bacteria under anaerobic conditions in which pathogenicity is exerted.

Further, at this time, by measuring a response using a diagnosing apparatus similar to that described above (Fig. 6), it has been found that electrochemical properties are specifically expressed under anaerobic conditions and the presence of pathogenic bacteria can be easily detected.

The present inventors completed the present invention with the idea of being capable of accurately detecting the presence of "pathogenic (having pathogenicity)" microbes (typically bacteria) contained in the sample solution by measuring the above-mentioned response. That is, the present invention has been completed with the idea that whether or not pathogenic microbes, of microbes responsible for an illness in the oral cavity, which are each a type of a so-called opportunistic bacterium, are present in the sample solution can be detected by measuring an electrochemical response.

The existing caries detection method and diagnosing apparatus (JP 2012 024085 A) have been used to detect the above-mentioned caries-causing microbes themselves and/or the metabolites thereof, or the like. However, the microbes responsible for caries are known as so-called opportunistic bacteria, and presumed to be present also in the oral cavity of a healthy person. The existing caries detection method does not take into account at all whether or not the bacteria are present in the oral cavity of a subject in a caries-causing (pathogenic) state. The present inventors have presumed that this is a cause of the obtained results being inaccurate.

In the diagnosing apparatus according to the embodiment of the present invention, electric characteristics newly found by the present inventors that microbes according to an illness in the oral cavity express electron-transfer enzymes specifically in the pathogenic state and electric current generation is observed accordingly as described above are used as measuring principles. Therefore, information for determining a state of progress of an illness in the oral cavity of a subject can be provided more accurately and easily.

Note that the cases where body fluid is saliva and the illness is caries and a periodontal disease have been described above, but the diagnosing apparatus according to the embodiment of the present invention is not limited to the above description and can be applied to the illness caused by microbes (particularly bacteria) regardless of the type of the body fluid and the type of the illness.

The body fluid applicable to the diagnosing apparatus according to the embodiment of the present invention is not particularly limited. Examples thereof include blood, lymph fluid, tissue fluid, body cavity fluid, digestive fluid, sweat, tears, runny nose, urine, semen, vaginal fluid, amniotic fluid, and milk.

Further, examples of the illness include, but are particularly not limited to, a eubacterial infection and a fungal infection.

### <Sample solution>

The sample solution applicable to the diagnosing apparatus according to the embodiment of the present invention is not particularly limited as long as it contains body fluid of a subject. The body fluid is as described above.

The method of obtaining the sample solution is not particularly limited. Examples of the method include, but are not particularly limited to, directly collecting body fluid of a subject collecting body fluid from a member wiping off the body of a subject, and washing the body of a subject with a buffer to collect the cleaning liquid.

Among them, saliva is favorable as the body fluid. The saliva contains bacteria in the oral cavity of a subject. In accordance with the diagnosing apparatus according to this embodiment, it is possible to provide information for determining the presence or absence of bacteria in a pathogenic state and the growth state of the bacteria in the oral cavity of a subject. As a result, it is possible to provide information for determining the type of an illness that has developed in the oral cavity and a state of progress of the illness.

In the case where the sample solution contains saliva, the subject is not particularly limited. As the subject, an animal having a tooth (particularly a mammal) is favorable, and a human is favorable. Examples of mammals other than a human include, but are not limited to, dogs, cats, horses, cows, pigs, sheep, goats, donkeys, mules, camels, llamas, alpacas, tortoises, zebus, yaks, guinea pigs, rabbits, foxes, fennec foxes, and monkeys.

The sample solution only needs to contain saliva and may contain components other than saliva. Examples of the components other than saliva include a solvent (water and/or an organic solvent) and an additive. The additive is not particularly limited. Examples of the additive include organic substances (e.g., glucose, yeast extract, etc.), which are utilized in microbes.

The sample solution 102 is introduced into the cell 101 so as to come into contact with the first electrode 103 and the second electrode 104 in the diagnosing apparatus 100. The method of introducing the sample solution 102 into the cell 101 is not particularly limited. The sample solution may be introduced directly from the oral cavity of a subject, or a sample solution prepared by adding a solvent and/or an additive to saliva obtained from the oral cavity of a subject as necessary may be introduced.

The voltage applying unit 105 is capable of controlling a voltage value applied between the first electrode 103 and the second electrode 104 (referred to also as "between electrodes"), timing, and the like.

In the diagnosing apparatus 100, the above-mentioned voltage applying unit 105 is part of the potentiostat 107, but the diagnosing apparatus according to the embodiment of the present invention is not particularly limited. The voltage applying unit 105 only needs to be capable of applying a desired voltage between the first electrode 103 and the second electrode 104.

The operator (who may be an operator other than a subject, or the subject himself/herself) of the diagnosing apparatus operates the potentiostat 107 to apply a voltage between the electrodes by the voltage applying unit 105. At this time, the voltage applied between the electrodes may be constant (a constant voltage), or may be increased or decreased in accordance with the time of application. In the case of increasing or decreasing the voltage to be applied, the voltage may be changed in a stepwise manner in accordance with the time of voltage application, or the voltage may be changed in a continuous manner in accordance with the time of voltage application. That is, the voltage to be applied may have a predetermined value (constant value), may be a sweep voltage, or may be a combination thereof.

In the diagnosing apparatus 100, an operator operates the potentiostat 107 to apply a voltage between the electrodes by the voltage applying unit 105, but the diagnosing apparatus according to the embodiment of the present invention is not limited to the above. For example, a potentiostat (as a result, a voltage applying unit) may be controlled from a control unit of the terminal 110 connected to the potentiostat so as to be capable of performing data communication with each other. In this case, the operator is capable of applying a voltage between the electrodes by operating a terminal.

Further, as described below (e.g., a diagnosing apparatus according to a second embodiment), in the case of a diagnosing apparatus where a voltage applying unit and a control unit are integrally formed, a voltage may be applied by operating the diagnosing apparatus itself.

When a voltage is applied between the electrodes, in the case where pathogenic microbes (typically pathogenic bacteria) are present in the sample solution, a metabolic current due to oxidation of organic substances and/or an oxidation-reduction current due to electron-transfer enzymes expressed in the extracellular membrane are generated, and the above-mentioned response is detected in the measuring unit. According to the claimed invention, the electrochemical response comprises, in the case where pathogenic microbes are present in the sample solution, the metabolic current due to oxidation of organic substances.

It is generally known that saliva contains 10⁸ to 10¹¹ CFU/ml of 100 types or more of bacteria. In these bacteria, bacteria responsible for an illness and bacteria that do not cause an illness are mixed, and pathogenic and non-pathogenic bacteria are mixed even in such bacteria responsible for an illness.

In accordance with a diagnosing apparatus according to an embodiment of the present invention, in the case where bacteria responsible for an illness in the oral cavity are present in a pathogenic state among the above-mentioned bacteria, it is possible to provide information for specifically detecting the above-mentioned current value and determining a state of progress of the illness in the oral cavity of a subject.

### <Electrode>

There is no particular limitation on the material of the electrode, and a known electrode material can be used. Examples of the electrode material include carbon, gold, platinum, silver, molybdenum, cobalt, nickel, palladium, and ruthenium. Indium tin oxide or the like may be used, and a known material for an electrode can be used.

Further, there is no particular limitation on the shapes and the like, and a known electrode for an electrochemical measurement cell can be used. Among them, it is favorable that the area of the electrode in contact with the sample solution is 1 cm² or less because even a smaller sample solution (specifically, 0.1 to 5 ml) can be detected sensitively.

### <Cell>

The cell is not particularly limited, and a cell known for electrochemical measurement can be used. The cell is favorably made of an insulating material. For example, the cell is formed of an insulating material, e.g., a thermoplastic resin such as polyetherimide (PEI), polyethylene terephthalate (PET), and polyethylene (PE), a thermosetting resin such as a polyimide resin and an epoxy resin, glass, ceramic, or paper. Although there is no particular limitation on the size of the cell, the size can be appropriately selected in accordance with the amount of the sample solution to be used, and is favorably a volume of approximately 0.1 to 5 ml.

Further, the cell may be configured to be hermetically sealable. In the case where the cell is hermetically configured, more sensible measurement can be performed in some cases. There is no particular limitation on the method of hermetically configuring the cell, and a known method can be applied. Examples thereof include a method of using a cell with a lid, which includes a cell and a lid portion covering an opening of the cell.

The measured response is compared with a reference in the analysis output unit 109 by using a numerical value obtained from the response. As a result, information for determining at least one item selected from the group consisting of the type of an illness that has developed in the oral cavity of a subject and a state of progress of the illness is provided (output to the outside) from the analysis output unit 109.

Here, the reference is a reference defining a relationship between a numerical value obtained from an electrochemical response and at least one item selected from the group consisting of the type of the illness that has developed and a state of progress of the illness, and is stored in the storage unit 108 in advance.

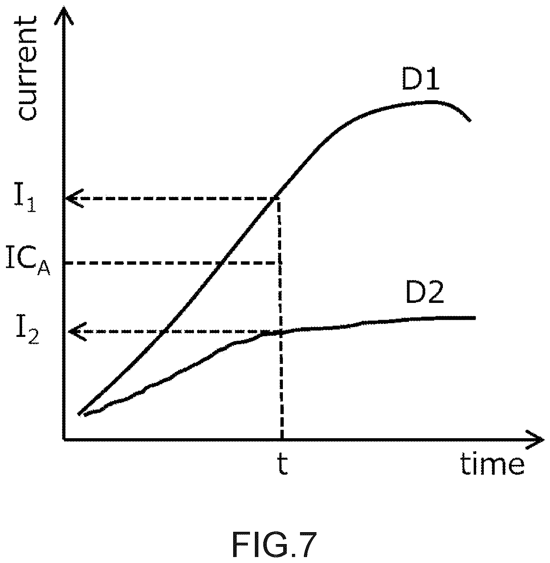

Fig. 7 is an explanatory diagram for describing an example of the above-mentioned method of performing comparison by the analysis output unit. Fig. 7 is a schematic graph showing the relationship (electric response) between current values obtained from a sample solution D1 and a sample solution D2 and the time of voltage application (measurement time) in the case where a predetermined voltage is applied between the electrodes (constant potential measurement).

The reference in Fig. 7 is the magnitude of the current value at a predetermined measurement time, and is stored in the storage unit 108 as a predetermined value IC_{A} by machine-learning or the like in relation to the presence or absence of the development of an illness (typically caries) in the oral cavity of a subject.

Here, in the case where the current value detected at a time t is I₁ when the sample solution D1 is measured, the analysis output unit compares this value with IC_{A} and outputs an indication that there is a possibility that an illness (typically caries) has developed in the oral cavity of the subject.

Note that the output content may be, for example, the value itself of the difference between I₁ and IC_{A}, or both the value of the difference and information relating to the possibility of the development of an illness. Further, I₁ and/or the value of the difference between I₁ and IC_{A} can be used also as diagnostic markers for an illness.

Meanwhile, in the case where the current value detected at the time t is I₂ when the sample solution D2 is measured, the analysis output unit compares this value with IC_{A} and outputs an indication that there is no possibility of an illness that has developed in the oral cavity of the subject. The output content in this case is similar to that described above.

Note that although the reference in Fig. 7 is the magnitude of the current value at a predetermined measurement time, the reference in the diagnosing apparatus according to the embodiment of the present invention is not limited to the above, and only needs to be a numerical value obtained from the measured response. As such a numerical value, for example, the absolute value of the current, the rate of change of the current (dl/dt, first order differential value), the second order differential value (d²I/dt²), the inflection point of dl/dt (time until the rise of the current value), the maximum value of the current, the time until the maximum value of the current is obtained, or the like may be used.

The numerical value to be compared with the reference is favorably the absolute value of the current or the difference (e.g., the difference in the shape of the graph of time-current values) from the electrochemical response measured using body fluid (typically saliva) obtained from the oral cavity of at least one selected from the group consisting of a subject in a healthy state and a healthy person different from the subject.

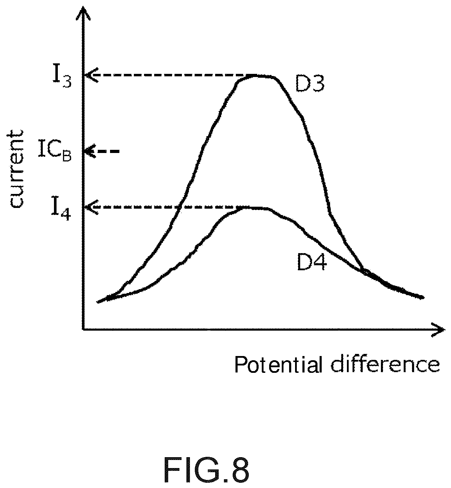

Further, as another example, a schematic graph showing the magnitude of the current value with respect to the potential difference between electrodes of a sample solution D3 and a sample solution D4 in the case where a sweep voltage is applied between the electrodes is shown in Fig. 8.

Here, in the case where the maximum value of the current detected for the sweep voltage is I₃ when the sample solution D3 is measured, the analysis output unit compares this value with a reference IC_{B} and outputs an indication that there is a possibility that an illness (typically caries) has developed in the oral cavity of the subject.

Note that the output content may be, for example, the value itself of the difference between I₃ and IC_{B}, or may be both the value of the difference and information regarding the possibility of the development of an illness. I₃ and/or the difference between I₃ and IC_{B} can be used as diagnostic markers for an illness.

Meanwhile, in the case where the maximum value of the current value detected for the sweep voltage is I₄ when the sample solution D4 is measured, the analysis output unit compares this value with IC_{B} and outputs an indication that there is no possibility of an illness that has developed in the oral cavity of the subject. The output content in this case is similar to that described above.

Note that the reference in Fig. 8 is the maximum value of the current obtained for the sweep voltage, but the reference in the diagnosing apparatus according to the embodiment of the present invention is not limited to the above, and may be, for example, the number of peaks and/or the widths of the peaks.

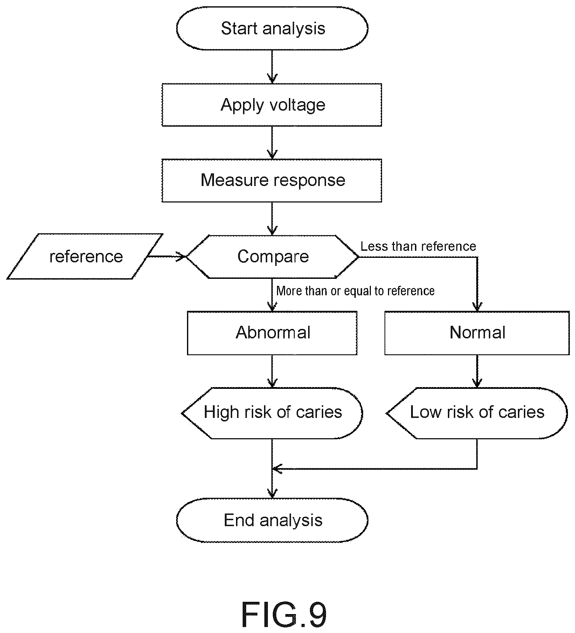

Fig. 9 shows an example of an analytical flow using the diagnosing apparatus according to the embodiment of the present invention. First, a sample containing saliva is introduced into a cell and the analysis is started. Next, a voltage is applied between the electrodes and the resulting electrochemical response (here, the current value) is measured. The resulting current value is compared with the reference, the current value is treated as an abnormal value in the case where the current value is greater than or equal to the reference, and "high risk of caries" indicating that there is a high possibility of the development of an illness in the oral cavity of a subject is output.

Meanwhile, the current value is treated as a normal value in the case where the current value is lower than the reference, "low risk of caries" indicating that the risk of an illness in the oral cavity of a subject is low is output, and the analysis is completed.

Note that "high risk of caries" and "low risk of caries" are described as output content in Fig. 9, but the diagnosing apparatus according to the embodiment of the present invention is not particularly limited. Indications that caries is absent (or has not developed) in the case of a normal value and caries is present (or has developed) in the case of an abnormal value may be shown by output other than the above. Further, the obtained current value itself and/or the relationship between the reference and the measured value (typically, the difference between the reference and the measured value) may be shown.

Further, the value greater than or equal to the reference is treated as the abnormal value when performing comparison with the reference value in the above-mentioned embodiment, but the value exceeding the reference may be diagnosed as the abnormal value. In such a case, it is favorable to diagnose the value lower than or equal to the reference as the normal value.

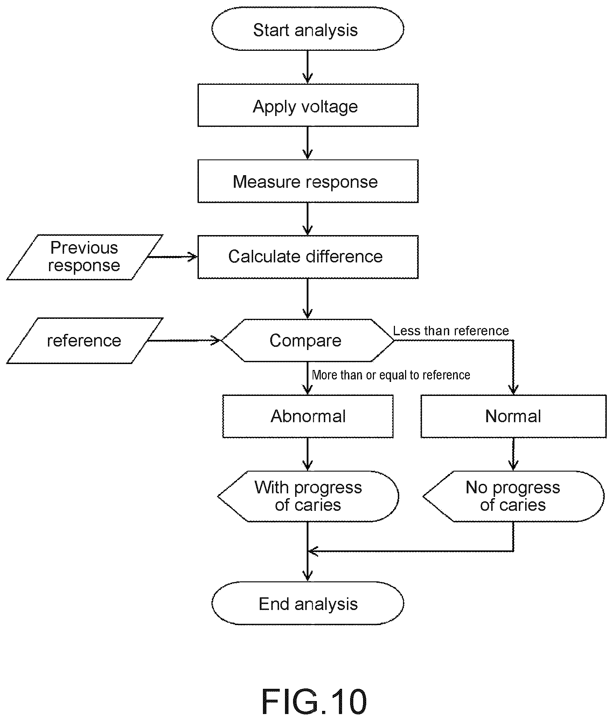

Fig. 10 shows an example of another analytical flow using the diagnosing apparatus according to the embodiment of the present invention. First, a sample containing saliva is introduced into a cell and diagnosis is started. Next, a voltage is applied between the electrodes and the resulting electrochemical response (here, the current value) is measured. Next, the difference between the obtained current value and a previous measurement value recorded in the storage unit is calculated.

It is favorable that the previous measurement value is a measurement value of a different sample solution, which contains saliva obtained at another time from the same subject. For example, it is favorable that the value is a value measured for a sample solution containing saliva obtained from the same subject at substantially the same time of the previous day.

Next, the resulting difference is compared with the reference, and is treated as an abnormal value in the case where it is greater than or equal to the reference.

In this case, the difference between the current value (previous current value) measured previously for another sample solution containing saliva obtained from the same subject and the current value obtained this time is calculated, and can be compared with the reference to provide information for determining the presence or absence of progress of an illness (typically caries) in the oral cavity.

That is, in the case where the difference between the current values and the reference is equal to or greater than the reference, there is a possibility that an illness (typically caries) has progressed, and "With progress of caries" is output. Meanwhile, in the case where the difference is lower than the reference, the value is treated as a normal value, it is highly likely that caries has not progressed in the oral cavity of the subject, and therefore, "no progress of caries" is output. Thus, the analysis is completed.

Note that "with progress of caries" and "no progress of caries" are described as the output content in Fig. 10, but the output content according to the diagnosing apparatus according to the embodiment of the present invention is not particularly limited. Indications that caries has not progressed in the case of a normal value and caries has progressed in the case of an abnormal value may be shown by output other than the above.

Further, in the above-mentioned embodiment, for example, in the case where the current value under a particular electrode condition is compared with the reference, the value greater than or equal to the reference is treated as an abnormal value, but the value exceeding the reference may be diagnosed as an abnormal value. In this case, it is favorable to diagnose the value lower than or equal to the reference as a normal value.

Conventionally, it is determined when a dentist visually recognizes a black spot in the enamel of a target tooth of a subject (patient) that caries has been generated, and treatment of removing the part where caries has been generated, or the like has been performed.

In accordance with the diagnosing apparatus according to the embodiment of the present invention, since the possibility of generation of caries can be detected at a stage before a black spot is observed, the advantage that the subsequent treatment can be less severe is provided.

### [Second embodiment of diagnosing apparatus]

A diagnosing apparatus according to a second embodiment of the present invention falling under the claimed invention is a diagnosing apparatus including: a cartridge type biosensor including a first electrode, a second electrode, and a cell; and a measuring device including a voltage applying unit, a measuring unit, a storage unit, an analysis output unit, and a control unit that controls the respective units described above, in which the electrodes of the biosensor are electrically connectable to the voltage applying unit and the measuring unit of the measuring device.

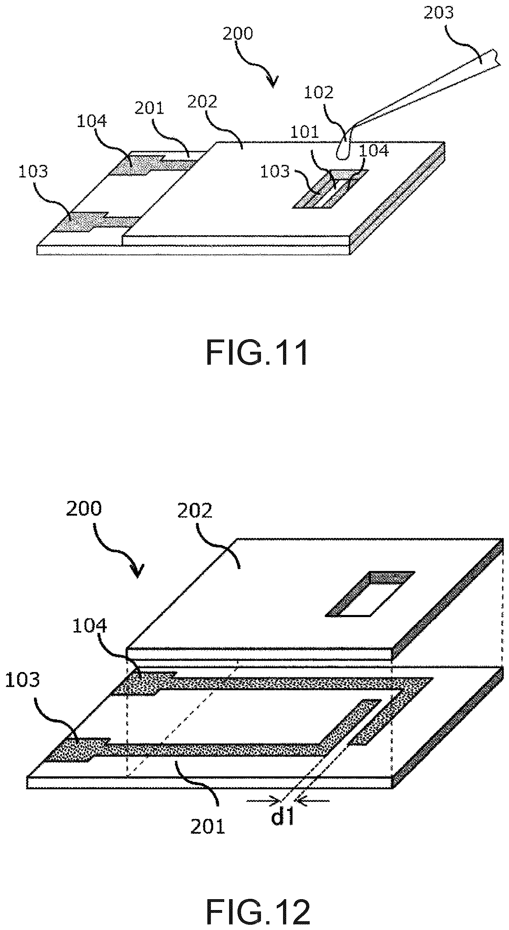

Fig. 11 is a perspective view showing a biosensor of a diagnosing apparatus according to the second embodiment of the present invention falling under the claimed invention.

In a biosensor 200 in Fig. 11, a film-like first electrode 103 and a film-like second electrode 104 are disposed on a lower insulating substrate 201.

The biosensor 200 includes the cell 101 formed by an upper insulating substrate 202 and the lower insulating substrate 201, and the sample solution 102 can be added thereto dropwise by a pipette 203.

Note that the sample solution may be added dropwise by those other than the pipette.

Fig. 12 is a perspective view showing a state in which the upper insulating substrate 202 of the biosensor 200 is separated. The first electrode 103 and the second electrode 104 in the biosensor 200 can be formed using, for example, a known photolithography technology or a known printing technology.

The shortest distance between the first electrode 103 and the second electrode 104 in the biosensor 200, i.e., a distance d1 between the two electrodes in the part where the first electrode 103 and the second electrode 104 are closest to each other, is not particularly limited, but is favorably 10 to 3,000 nm in terms of achieving a diagnosing apparatus having a faster response rate.

When the shortest distance d1 between the first electrode 103 and the second electrode 104 is within the above-mentioned range, a current flows between the electrodes having a potential difference when bacteria responsible for an illness in the oral cavity, which are in a pathogenic state, bridge the electrodes. That is, when bacteria in the pathogenic state are present in the sample solution, the bacteria can be detected immediately by detecting the above-mentioned current value.

Specifically, it is presumed that a current value of approximately 100 pA can be measured when one of the above-mentioned bacteria bridges the electrodes having a potential difference of 50 mV.

In accordance with the diagnosing apparatus according to the above-mentioned embodiment, it is possible to achieve an effect that the time from introducing a sample solution into a cell to detecting the current becomes shorter.

The shortest distance d1 can be appropriately selected in accordance with the size of the bacterium to be detected. Above all, in the case where the distance d1 is a distance at which one of the cells of the microbe (typically the bacterium) responsible for an illness can be short-circuited, it is favorable because information for determining a type of an illness that has developed in a subject in the above-mentioned diagnosing apparatus can be easily and quickly provided.

For example, a case where the distance d1 is 700 nm to 2,000 nm will be described.

Assumption is made that a sample solution containing saliva contains bacteria responsible for caries (e.g., Streptococcus mutans) and bacteria responsible for a periodontal disease (e.g., Porphyromonas gingivalis (hereinafter, PG bacteria) or Aggregatibacter actinomycetem comitans (hereinafter, AA bacteria)).

At this time, the bacteria responsible for caries are Streptococcus sp. and the size of one cell thereof is approximately 400 to 500 nm in diameter. Meanwhile, PG bacteria and AA bacteria are anaerobic bacilli and facultative anaerobic bacilli, and the size of one cell thereof is approximately 1 to 2 µm.

At this time, when the distance d1 is 700 nm to 2,000 nm, in the case where bacteria responsible for a periodontal disease are present in a sample solution, the above-mentioned bacteria adhere between the electrodes, and the electron-transfer enzymes expressed in the extracellular membrane cause the electrodes to be short-circuited, which can be selectively detected. Meanwhile, the size of each of the caries-causing bacteria is less than the distance d1 between the electrodes, and therefore, they are not detected instantaneously.

As described above, by setting the distance d1 in accordance with the size of each of the cells of the bacteria to be measured, the bacteria to be measured can be selectively, easily, and quickly measured.

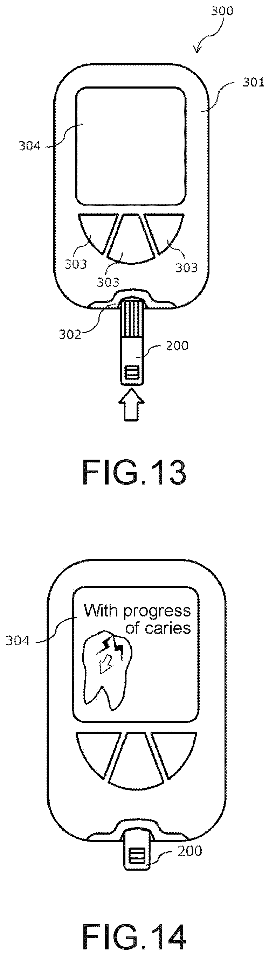

Fig. 13 is a top view of a diagnosing apparatus 300 according to this embodiment. The diagnosing apparatus 300 includes the biosensor 200 described above and a measuring device 301, and is used by inserting the biosensor 200 into a sensor insertion hole 302 of the measuring device 301. When the biosensor 200 is inserted into the sensor insertion hole 302, a measuring unit and a voltage applying unit (not shown) in the measuring device 301 are electrically connected to electrodes of the biosensor 200, and the measuring device 301 can be used.

In accordance with the diagnosing apparatus according to this embodiment, the biosensor 200 can be replaced for each sample solution, and analysis can be performed in a state in which the inside of the cell including the electrode surface is clean. This makes it possible to provide more accurate results.

The measuring device 301 includes an operation button 303 and a display unit 304 on a casing. The operator of the diagnosing apparatus 300 is capable of performing measurement by adding a sample solution to a cell of the biosensor 200 dropwise, inserting the biosensor 200 into the sensor insertion hole 302 of the measuring device 301, and then operating the operation button 303 in accordance with the displayed content of the display unit 304.

Note that the operation button 303 can be used for operations such as various types of setting relating to the measuring device, starting diagnosis, and finishing the diagnosis. The measuring device may include a touch panel that can be used for the above-mentioned operations together with or instead of the operation button.

Further, the display unit 304 is used to display information provided from the analysis output unit of the measuring device 301, setting information relating to diagnosis, a state of progress of measurement, and the like. The display unit 304 may include a liquid crystal panel, a plasma display panel, an electroluminescent panel, or the like. Further, in the case where the display unit includes a touch panel, the display unit may be configured to operate the measuring device, i.e., the operation button and the display unit may be integrally formed.

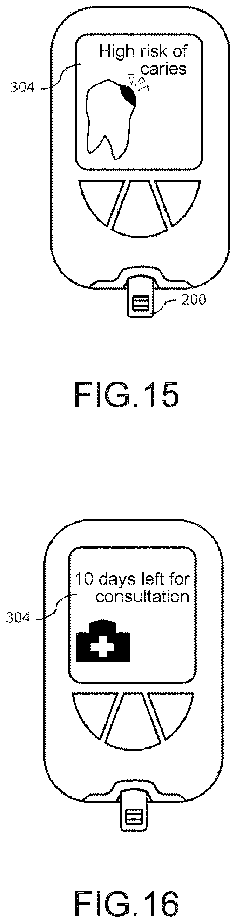

For example, in the case where the current value in the sample solution is greater than or equal to the reference by the above-described analytical flow, "high risk of caries" is displayed on the display unit 304 (Fig. 15). Further, in the case where the current value is greater than or equal to the reference as compared with the previous current value, "with progress of caries" (Fig. 14) is displayed. Further, a recommended consultation deadline to a dental clinic can be displayed, as described below (Fig. 16).

Note that the measuring device 301 includes a measuring unit, a voltage applying unit, a storage unit, an analysis output unit, and a control unit that controls the respective units (which are not shown), and the control unit is a processor. Examples of the control unit include, but are not limited to, a central processing unit (CPU), a microprocessor, a processor core, a multiprocessor, an ASIC (application-specific integrated circuit), and an FPGA (field programmable gate array).

Further, the control unit reads the program stored in the storage unit and performs predetermined arithmetic processing in accordance with the program.

Further, the control unit is capable of writing and reading the operation result according to the program to/from the storage unit as appropriate.

Further, the storage function of the storage unit can be realized by a non-volatile memory such as an HDD (hard disk drive) and an SSD (solid state drive). Further, the storage unit may have a function as a memory for writing or reading intermediate progress or the like of the arithmetic processing by the control unit. The memory function of the storage unit can be realized by a volatile memory such as a RAM (Random Access memory) and a DRAM (Dynamic Random Access memory).

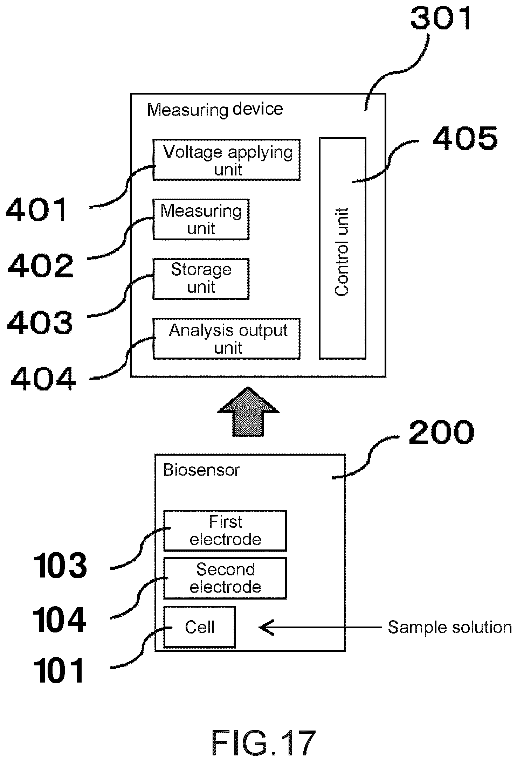

Fig. 17 is a diagram describing a functional configuration of a diagnosing apparatus including the measuring device 301 and the biosensor 200. The measuring device 301 includes a voltage applying unit 401, a measuring unit 402, a storage unit 403, an analysis output unit 404, and a control unit 405 that is a processor controlling the respective units described above.

Further, the biosensor 200 includes the first electrode 103, the second electrode 104, and the cell 101.

First, a sample solution is introduced into the cell 101 so as to come into contact with the first electrode 103 and the second electrode 104. Next, the biosensor 200 and the measuring device 301 are connected to each other, and the first electrode 103 and the second electrode 104 are electrically connected to the voltage applying unit 401 and the measuring unit 402.

Next, the voltage applying unit 401 controlled by the control unit 405 applies a voltage between the first electrode 103 and the second electrode 104, and the measuring unit 402 measures the current value at this time. Next, the analysis output unit 404 controlled by the control unit 405 compares the reference stored in advance in the storage unit 403 with the obtained current value, and outputs information relating to a state of progress of an illness in the oral cavity of a subject, and this is displayed on the display unit that has been described above. Note that the reference is as described above in the description of the diagnosing apparatus according to the first embodiment of the present invention.

In the diagnosing apparatus according to the above-mentioned embodiment, the biosensor is of a cartridge type, and the biosensor may be updated for each sample solution. By doing so, it becomes easier to prevent contamination between different sample solutions on the electrodes.

### (Another embodiment of biosensor)

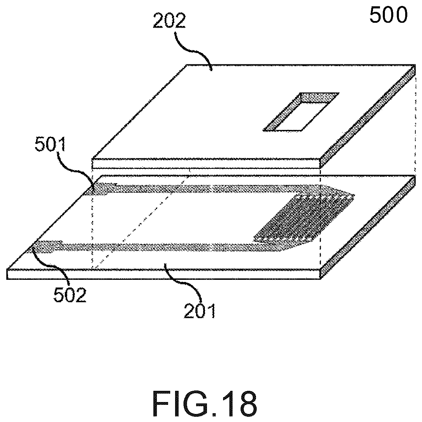

Fig. 18 is a top view showing another embodiment of a biosensor that can be used for the diagnosing apparatus according to the embodiment of the present invention. A biosensor 500 includes a comb-shaped first electrode 501 and a comb-shaped second electrode 502 disposed on the lower insulating substrate 201. A cell is formed by the lower insulating substrate 201 and an opening of the upper insulating substrate 202.

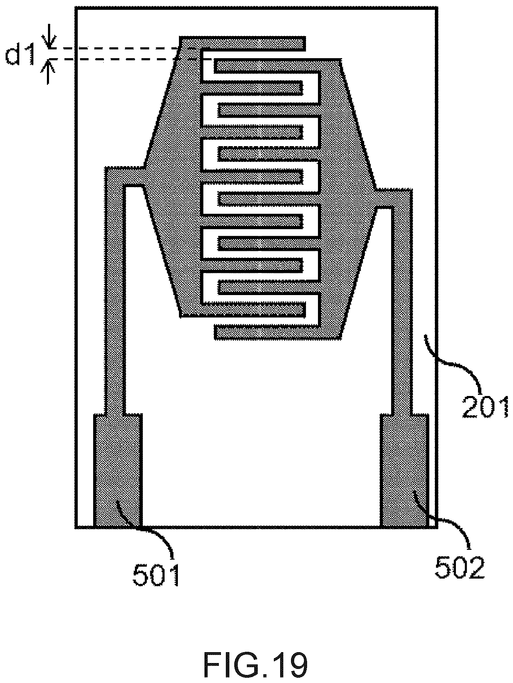

Fig. 19 is a top view schematically showing a state in which the upper insulating substrate 202 of the biosensor 500 has been removed. The comb-shaped first electrode 501 and the comb-shaped second electrode 502 are comb-shaped electrodes corresponding to each other. Note that although both the first electrode and the second electrode are comb-shaped electrodes in the biosensor according to this embodiment, but a response tends to be faster as described above when at least one selected from the group consisting of the first electrode and the second electrode is a comb-shaped electrode.

At this time, the embodiment of the distance d1 is as described above.

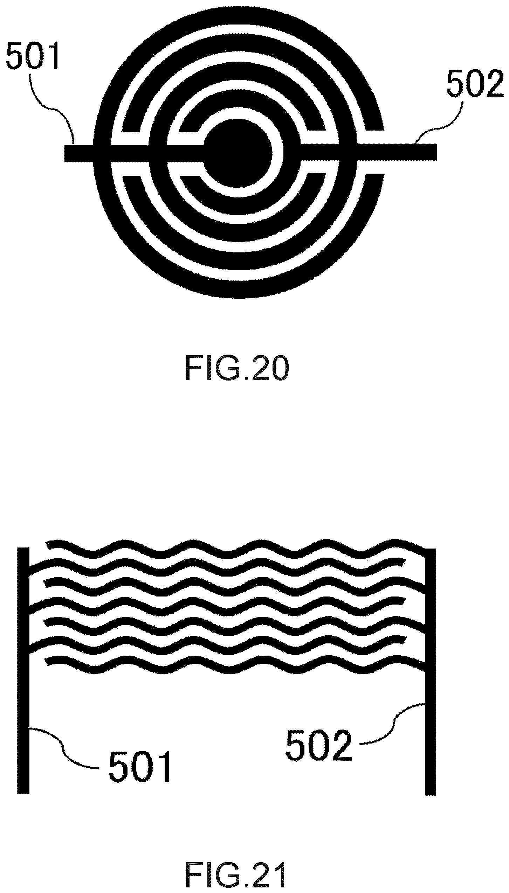

Note that as used herein, a comb-shaped electrode means an electrode configured such that two facing electrodes are combined with each other. Note that the structure of the comb-shaped electrode may be a so-called comb-shape, a concentric shape as shown in Fig. 20, and may be a wave shape as shown in Fig. 21.

### [Third embodiment of diagnosing apparatus]

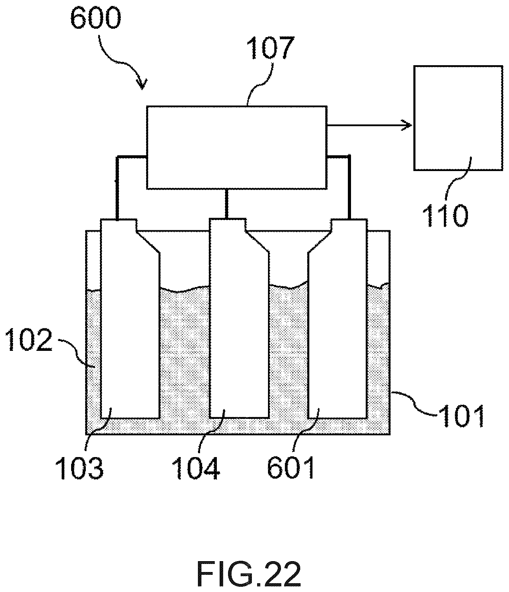

Fig. 22 is a schematic diagram showing a diagnosing apparatus according to a third embodiment of the present invention falling under the claimed invention. A diagnosing apparatus 600 is similar to the diagnosing apparatus according to the first embodiment, which has been described above, except that it further includes a reference electrode 601 in the cell 101 so as to come into contact with the sample solution 102.

Note that the first electrode 103 and the second electrode 104 are electrically connected to the reference electrode 601 via the potentiostat 107.

Note that the diagnosing apparatus 600 includes the first electrode 103, the second electrode 104, and the reference electrode 601, but the diagnosing apparatus according to the embodiment of the present invention is not limited to the above. The diagnosing apparatus may include a counter electrode instead of the reference electrode 601, or may include a first electrode and two reference electrodes, or a first electrode, a second electrode, and two or more reference electrodes.

Note that in the diagnosing apparatus including a reference electrode, the potential of the electrode can be measured, so that a diagnosing apparatus having better effects of the present invention can be achieved.

Note that as the reference electrode, a known reference electrode for an electrochemical measurement cell can be used, and for example, a silver/silver chloride electrode or the like can be used.

Further, as the counter electrode, a known counter electrode for an electrochemical measurement cell can be used.

### [Fourth embodiment of diagnosing apparatus]

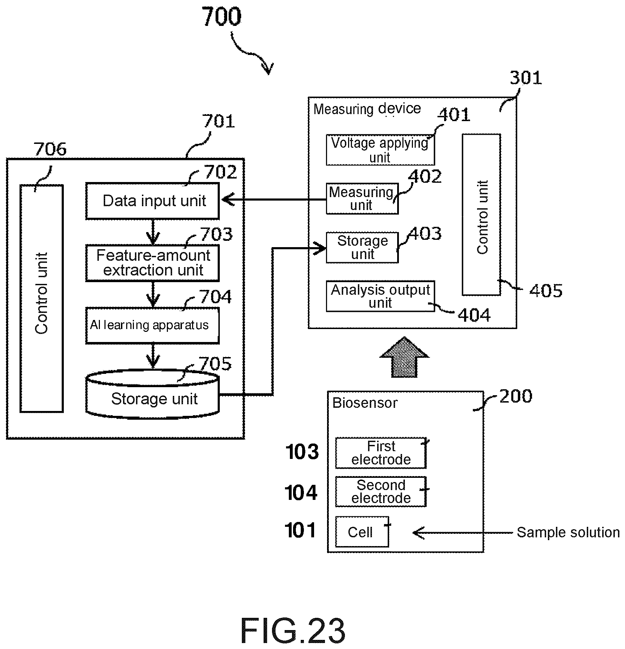

Fig. 23 is a functional explanatory diagram of a diagnosing apparatus 700 according to a fourth embodiment of the present invention falling under the claimed invention, including the measuring device 301, the biosensor 200, and a reference creating device 701 configured to create a reference by machine-learning on the basis of the electrochemical response.

The diagnosing apparatus 700 includes the biosensor 200, the measuring device 301, and the reference creating device 701, and the measuring device 301 and the reference creating device 701 are connected so as to be capable of performing data communication with each other.

The reference creating device 701 includes a data input unit 702 that inputs time-varying data of a current value, a feature-amount extraction unit 703 that extracts a feature amount from the input current value, an Al learning apparatus 704 that calculates a reference by machine-learning on the basis of the feature amount, a storage unit 705, and a control unit 706 that is a processor that controls the respective units described above.

The control unit 706 is a processor having a function that controls the respective units of the reference creating device 701. Examples of the control unit 706 include, but are not limited to, a central processing unit (CPU), a microprocessor, a processor core, a multiprocessor, an ASIC (application-specific integrated circuit), and an FPGA (field programmable gate array).

Further, the control unit 706 reads the program stored in the storage unit 705 and performs predetermined arithmetic processing in accordance with the program.

Further, the control unit is capable of writing and reading the operation result according to the program to/from the storage unit 705 as appropriate.

Further, the storage function of the storage unit 705 can be realized by a non-volatile memory such as an HDD (hard disk drive) and an SSD (solid state drive). Further, the storage unit may have a function as a memory for writing or reading intermediate progress of the arithmetic processing by the control unit. The memory function of the storage unit can be realized by a volatile memory such as a RAM (Random Access memory) and a DRAM (Dynamic Random Access memory).

Under the control of the control unit 405, the measuring device 301 transmits data (response) relating to the temporal change of the current value relating to a sample solution to the reference creating device 701.

The feature-amount extraction unit 703 extracts information such as an absolute value of the current, a first derivative value of the time, a second derivative value of the time, the time until the rise of the current value, the maximum current value, the time until the maximum current value, and the rising method of the current value from the temporal change (response) of the input current value, and transmits the extracted information (feature amount) to the Al learning apparatus 704.

The Al learning apparatus 704 performs machine-learning on the basis of the feature amount provided from the feature-amount extraction unit 703 and the information relating to the type of an illness and/or a state of progress of the illness. The Al learning apparatus 704 creates a reference defining the relationship between a numerical value obtained from the response as a result of the machine-learning and at least one item selected from the group consisting of the type of an illness and a state of progress of the illness.

The method of machine learning is not particularly limited, and a known method such as a neural network, discriminant analysis, logistic regression analysis, genetic programming, inductive logic programming, support vector machine, and clustering only needs to be used.

Note that the control unit 706 reads the program stored in the storage unit 705 and performs predetermined arithmetic processing in accordance with the program.

Further, the control unit 706 is capable of writing or reading the operation result according to the program to/from the storage unit 705 as appropriate.

Further, the storage function of the storage unit 705 can be realized by a non-volatile memory such as an HDD (hard disk drive) and an SSD (solid state drive). Further, the storage unit 705 may have a function as a memory for writing or reading intermediate progress of the arithmetic processing by the control unit 706. The memory function of the storage unit can be realized by a volatile memory such as a RAM (Random Access memory) and a DRAM (Dynamic Random Access memory).

The reference stored in the storage unit 705 is transmitted to the measuring device 301 by the control unit 706. The measuring device 301 receives the above-mentioned reference, and the received reference is recorded on the storage unit 403 of the measuring device 301 and used for analyses.

### [Fifth embodiment of diagnosing apparatus]

A diagnosing apparatus according to a fifth embodiment of the present invention falling under the claimed invention is a diagnosing apparatus further including: a transmission unit configured to transmit the electrochemical response to an external server via a communication line; and a reception unit configured to receive at least one item selected from the group consisting of a consultation deadline and expected progress of the illness that has been calculated by machine-learning in the external server, on the basis of the electrochemical response transmitted from the transmission unit.

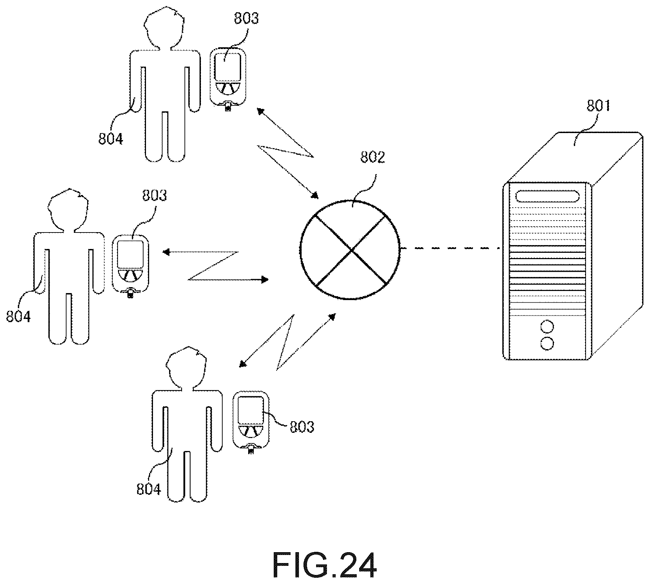

Fig. 24 shows a schematic diagram of a system including a diagnosing apparatus according to the above-mentioned embodiment. A diagnosing apparatus 803 transmits a response (including the feature amount that has been described above) obtained from body fluid of each subject 804 to a server 801 via a network (e.g., the internet) 802. In the server 801, at least one item selected from the group consisting of the consultation deadline and the expected progress of the illness is calculated by the machine-learning that has been described above.

In the above-mentioned server 801, data (response) of others is accumulated, the Al picks up data of a person who shows behavior close to that of the subject, and expectation data relating to the response change for the following days (e.g., increase in the current value) is calculated (expected progress of the illness). Further, also the deadline for visiting the medical clinic (which is defined by how many days left until the reference will be exceeded; the consultation deadline) is calculated.

The diagnosing apparatus 803 receives, from the server 801, at least one item selected from the group consisting of a consultation deadline and expected progress of the illness, and displays it.

### [Program (First embodiment)]

A program according to a first embodiment thereof, the program according to an embodiment of the present invention falling under the claimed invention, is a program that causes, in a system that includes the above-mentioned diagnosing apparatus and a server that is a computer, the diagnosing apparatus and the server being configured to communicate with each other via a network, the diagnosing apparatus including further a display unit, the computer to execute the procedures of: receiving a specific response determined to be abnormal by comparing the numerical value and the reference, of the electrochemical response obtained by the diagnosing apparatus; applying the specific response to an estimation model to estimate generation of the pathogenic microbes in the sample solution, the estimation model being learned to estimate, when an electrochemical response is input, presence or absence of generation of the pathogenic microbes responsible for the illness, learning having been performed in advance in the estimation model by learning data including an electrochemical response of a learning sample solution different from the sample solution and a microbiota analysis result of the learning sample solution; and prompting, where generation of the pathogenic microbes is expected in the sample solution, the display unit to display that microbiota analysis is necessary for the sample solution.

### <System>

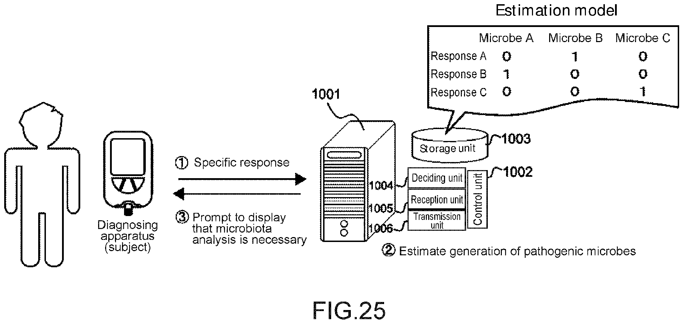

The above-mentioned system is a system including the diagnosing apparatus that has been described above and a server that is a computer, the diagnosing apparatus and the server being configured to be capable of communicating with each other via a network. Fig. 25 shows a schematic diagram showing the system.

In Fig. 25, a server 1001 is a computer that executes a program and performs processing, and specifically includes a control unit 1002, a storage unit 1003, a deciding unit 1004, a reception unit 1005, and a transmission unit 1006.

The control unit 1002 is a processor having a function that controls the respective units of the computer. Examples of the control unit 1002 include, but are not limited to, a central processing unit (CPU), a microprocessor, a processor core, a multiprocessor, an ASIC (application-specific integrated circuit), and an FPGA (field programmable gate array).

Further, the control unit 1002 reads the program stored in the storage unit 1003 and performs predetermined arithmetic processing in accordance with the program.

Further, the control unit is capable of writing and reading the operation result according to the program to the storage unit 1003 as appropriate.

Further, the storage function of the storage unit 1003 can be realized by a non-volatile memory such as an HDD (hard disk drive) and an SSD (solid state drive). Further, the storage unit may have a function as a memory for writing or reading intermediate progress of the arithmetic processing by the control unit. The memory function of the storage unit can be realized by a volatile memory such as a RAM (Random Access memory) and a DRAM (Dynamic Random Access memory).

The transmission unit 1006 and the reception unit 1005 transmit/receive various types of data and the like to/from devices connected via a network such as the Internet. Specifically, they may be an I/F that transmits/receives data to/from the diagnosing apparatus by radio or the like.

### <Processing by server>

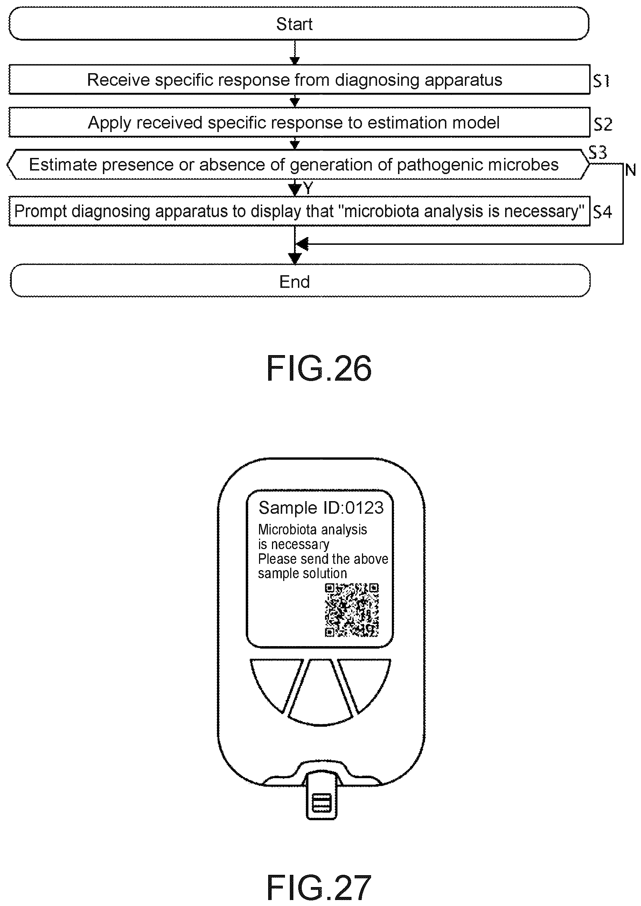

The above-mentioned program is a program executed by a server, and the process flow thereof is shown in Fig. 26.

First, in Step S1, the control unit of the server instructs the reception unit to receive, from the diagnosing apparatus, a specific response determined to be abnormal in the diagnosing apparatus, of an electrochemical response relating to a sample solution, and stores the data in the storage unit. The electrochemical response received from the diagnosing apparatus is as described above. Typical examples thereof include the relationship between the current value and the time of voltage application, and the relationship between the current value and the applied voltage.

Next, in Step S2, the control unit of the server applies the received electrochemical response to the estimation model stored in the storage unit. Learning has been performed in advance in the estimation model by learning data including an electrochemical response of a learning sample solution different from the sample solution and a microbiota analysis result of the learning sample solution.

Because the above-mentioned microbiota analysis result also includes information regarding whether or not the detected microbe corresponds to a pathogenic microbe responsible for an illness, inputting the electrochemical response of the sample solution to the above-mentioned estimation model can estimate the presence or absence of generation of pathogenic microbes (Step S3). In this embodiment, learning is performed in accordance with a learning model constructed by a neural network including a multi-layer neural network. The learning model constructed by the neural network including an input-layer, an output-layer, and an intermediate layer is capable of using any suitable method. For example, a CNN (Convolutional Neural Network) can also be applied.

Note that the above-mentioned Step S2 and Step S3 are executed by the control unit controlling the deciding unit and calling the estimation model stored in the storage unit.

Next, in the above-mentioned Step S3, in the case where the generation of pathogenic microbes is expected in the sample solution, the control unit controls the transmission unit to prompt the display unit of the diagnosing apparatus to display that microbiota analysis is necessary for the corresponding sample solution (Step S4).

Fig. 27 shows an example of displaying that microbiota analysis is necessary. In Fig. 27, a sample solution for which microbiota analysis is necessary is identified by the sample ID assigned each time of measurement, and an indication that microbiota analysis is necessary for the sample solution relating to the sample ID and a two-dimensional barcode (e.g., "QR Code (registered trademark)") in which the procedure of the microbiota analysis has been stored are displayed. Examples of the procedure of microbiota analysis include a method for transmitting a sample solution for microbiota analysis to an analysis institution having an analysis device, and a deadline for transmission.

### [Program (second embodiment)]

A program according to a second embodiment thereof, the program according to another embodiment of the present invention falling under the claimed invention, is a program that causes, in a system that includes the above-mentioned diagnosing apparatus, a server that is a computer, and an analysis device capable of performing microbiota analysis, the diagnosing apparatus, the server, and the analysis device being configured to communicate with each other via a network, the diagnosing apparatus including further a display unit, the computer to execute the procedures of: receiving a specific response determined to be abnormal by comparing the numerical value and the reference, of the electrochemical response obtained by the diagnosing apparatus, and a sample ID corresponding to the sample solution; applying the specific response to an estimation model to estimate generation of the pathogenic microbes in the sample solution, the estimation model being learned to estimate, when an electrochemical response is input, presence or absence of generation of the pathogenic microbes responsible for the illness, learning having been performed in advance in the estimation model by learning data including an electrochemical response of a learning sample solution different from the sample solution and a microbiota analysis result of the learning sample solution; prompting, where generation of the pathogenic microbes is expected in the sample solution, the display unit to display that microbiota analysis is necessary for the sample solution; receiving a result of a microbiota analysis of the sample solution and the sample ID from the analysis device; deciding presence or absence of the pathogenic microbes in the sample solution from the result of the microbiota analysis; and prompting, where it is decided from the result of the microbiota analysis that the pathogenic microbes have not been generated in the sample solution, the diagnosing apparatus to change the reference.

### <System>

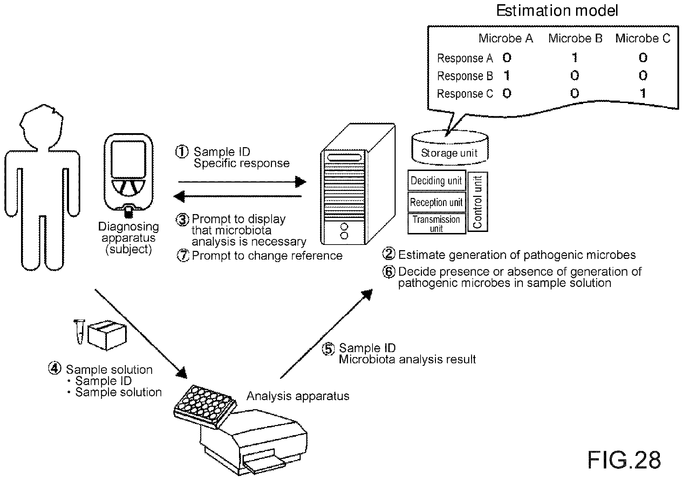

The above-mentioned system is a system including the diagnosing apparatus that has been described above, a server that is a computer, and an analysis device capable of performing microbiota analysis, the diagnosing apparatus, the server, and the analysis device being configured to be capable of communicating with each other via a network. Fig. 28 shows a schematic diagram of the system.

In Fig. 28, a diagnosing apparatus and a server are similar to the diagnosing apparatus and the server in the system to which the program according to the first embodiment thereof that has been described above is applied.

### <Processing by server>

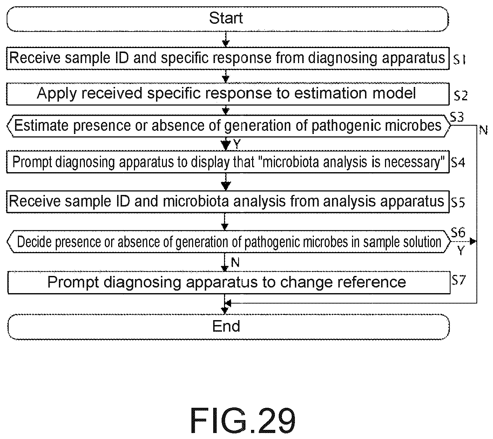

The above-mentioned program is a program executed by a server, and the process flow thereof is shown in Fig. 29.

First, in Step S1, the control unit of the server instructs the reception unit to receive, from the diagnosing apparatus, a specific response determined to be abnormal in the diagnosing apparatus, of an electrochemical response relating to a sample solution, and stores the data in the storage unit. The electrochemical response received from the diagnosing apparatus is as described above. Typical examples thereof include the relationship between the current value and the time of voltage application, and the relationship between the current value and the applied voltage.

Next, in Step S2, the control unit of the server applies the received electrochemical response to the estimation model stored in the storage unit, which has been described above. inputting the electrochemical response of the sample solution to the above-mentioned estimation model can estimate the presence or absence of generation of pathogenic microbes (Step S3).

Next, in the case where the generation of pathogenic microbes is expected in the sample solution in the above Step S3, the control unit controls the transmission unit to output, on the display unit of the diagnosing apparatus, an indication that microbiota analysis is necessary for the corresponding sample solution (Step S4).

Next, in Step S5, the control unit of the server controls the reception unit to receive, from the analysis device, the sample ID and the microbiota analysis result relating to the sample solution, and stores them in the storage unit. This sample ID is a sample ID commonly assigned to the sample solution whose response has been measured in the diagnosing apparatus and a sample solution for which microbiota analysis has been performed in the analysis device, and is one allocated by the control unit of the diagnosing apparatus as a value unique to the sample solution.

Next, in Step S6, the control unit of the server controls the deciding unit to determine the generation of the presence or absence of the pathogenic microbes in the sample solution from the microbiota analysis result.

As a result, in the case where it is determined that the pathogenic microbes have not been generated in the sample solution, the control unit controls the deciding unit to create a new reference that is determined to be normal for the above-mentioned specific response, and instructs the transmission unit to transmit the new reference and prompt the diagnosing apparatus to change the reference (Step S7).

At this time, the new reference does not necessarily need to be transmitted, and the control unit only needs to prompt the transmission unit to change the reference. In this case, a new reference is created in the diagnosing apparatus.

In accordance with the above-mentioned program, it is possible to update, in the case where a pathogenic microbe is not detected by microbiota analysis (false positive) in a response determined to be abnormal in the diagnosing apparatus, the reference of the diagnosing apparatus and cause the diagnosing apparatus to provide more accurate information.

### Reference Signs List

100: diagnosing apparatus
101: cell
102: sample solution
103: first electrode
104: second electrode
105: voltage applying unit
106: measuring unit
107: potentiostat
108: storage unit
109: analysis output unit
110: terminal
111: circuit
200: biosensor
201: lower insulating substrate
202: upper insulating substrate
203: pipette
300: diagnosing apparatus
301: measuring device
302: sensor insertion hole
303: operation button
304: display unit
401: voltage applying unit
402: measuring unit
403: storage unit
404: analysis output unit
405: control unit
500: biosensor
501: first electrode
502: second electrode
600: diagnosing apparatus
601: reference electrode
700: diagnosing apparatus
701: reference creating device
702: data input unit
703: feature-amount extraction unit
704: Al learning apparatus
705: storage unit
706: control unit
801: server
802: network
803: diagnosing apparatus
804: subject
1001: server
1002: control unit
1003: storage unit
1004: deciding unit
1005: reception unit
1006: transmission unit

## Claims

1. A diagnosing apparatus (100; 300; 600; 700; 803), comprising:
a cell (101) configured to introduce a sample solution (102) containing body fluid collected from a subject (804);
at least two electrodes (103, 104; 501, 502) that are disposed in the cell (101) and come into contact with the sample solution (102);
a voltage applying unit (105; 401) configured to apply a voltage between the electrodes (103, 104; 501, 502);
a measuring unit (106; 402) configured to measure an electrochemical response when the voltage is applied;
a storage unit (108; 403) configured to store a reference that defines a relationship between a numerical value obtained from the electrochemical response and at least one of a type of an illness and a state of progress of the illness; and
an analysis output unit (109; 404) configured to compare the numerical value obtained from the subject (804) with the reference, and provide information for determining at least one of the type of the illness that has developed in the subject (804) and the state of progress of the illness that has developed in the subject (804),
**characterized in that** the electrochemical response comprises, in the case where pathogenic microbes are present in the sample solution (102), a metabolic current due to oxidation of organic substances.

2. The diagnosing apparatus (100; 300; 600; 700; 803), according to claim 1, wherein the numerical value is an absolute value of a current measured by the measuring unit (106; 402) or a difference between the electrochemical response measured using body fluid obtained from the subject (804) and the electrochemical response measured using body fluid obtained from at least one of the subject (804) in a healthy state and a healthy person different from the subject (804).

3. The diagnosing apparatus (100; 300; 600; 700; 803), according to claim 1 or 2, wherein
the body fluid is saliva.

4. The diagnosing apparatus (100; 300; 600; 700; 803), according to claim 3, wherein
the illness is at least one of caries and a periodontal disease.

5. The diagnosing apparatus (100; 300; 600; 700; 803), according to any one of claims 1 to 4, wherein
the storage unit (108; 403) is further configured to record the measured electrochemical response, and
the analysis output unit (109; 404) is further configured to compare the electrochemical response obtained by measurement of the sample solution (102) with the electrochemical response that has been obtained by a preceding measurement prior to the measurement and recorded in the storage unit (108; 403), and provide information for determining the at least one of the type of the illness that has developed in the subject (804) and the state of progress of the illness that has developed in the subject (804) on a basis of the reference from a result of the comparison.

6. The diagnosing apparatus (100; 300; 600; 700; 803), according to any one of claims 1 to 5, wherein
the cell (101) is further configured to be hermetically sealable.

7. The diagnosing apparatus (300; 700; 803) according to any one of claims 1 to 6, wherein
a shortest distance between the electrodes (103, 104; 501, 502) in the cell (101) is a distance at which one of cells of a microbe responsible for the illness can be short-circuited.

8. The diagnosing apparatus (300; 700; 803) according to claim 7, wherein
the shortest distance between the electrodes (103, 104; 501, 502) in the cell (101) is 700 nm to 2,000 nm.

9. The diagnosing apparatus (700; 803) according to any one of claims 1 to 8, further comprising a reference creating device (701) configured to create the reference by machine-learning on a basis of the electrochemical response.

10. The diagnosing apparatus (803) according to any one of claims 1 to 9, further comprising:
a transmission unit configured to transmit the electrochemical response to an external server via a communication line; and
a reception unit configured to receive at least one of a consultation deadline and expected progress of the illness that has been calculated by machine-learning in the external server on a basis of the electrochemical response transmitted from the transmission unit.

11. An analysis method, comprising:
a step of introducing, into a cell (101) in which at least two electrodes (103, 104; 501, 502) are disposed, a sample solution (102) containing body fluid collected from a subject (804) so as to come into contact with the electrodes (103, 104; 501, 502);
a step of measuring an electrochemical response when a voltage is applied between the electrodes (103, 104; 501, 502); and
a step of comparing a reference defining a relationship between a numerical value obtained from the electrochemical response and at least one of a type of an illness and a state of progress of the illness with the numerical value obtained from the subject (804) to provide information for determining at least one of the type of the illness that has developed in the subject (804) and the state of progress of the illness that has developed in the subject (804),
**characterized in that** the electrochemical response comprises, in the case where pathogenic microbes are present in the sample solution (102),
a metabolic current due to oxidation of organic substances.

12. The analysis method according to claim 11, wherein
the body fluid is saliva.

13. The analysis method according to claim 12, wherein
the illness is at least one of caries and a periodontal disease.

14. A program that causes, in a system that includes a diagnosing apparatus (803) with the features of any one of claims 1 to 10 and a server (1001) that is a computer, the diagnosing apparatus (803) and the server (1001) being configured to communicate with each other via a network, the diagnosing apparatus (803) including further a display unit (304), the computer to execute the procedures of:
receiving a specific response determined to be abnormal by comparing the numerical value and the reference, of the electrochemical response obtained by the diagnosing apparatus (803);
applying the specific response to an estimation model to estimate generation of the pathogenic microbes in the sample solution (102), the estimation model being learned to estimate, when an electrochemical response is input, presence or absence of generation of the pathogenic microbes responsible for the illness, learning having been performed in advance in the estimation model by learning data including an electrochemical response of a learning sample solution different from the sample solution (102) and a microbiota analysis result of the learning sample solution; and
prompting, where generation of the pathogenic microbes is expected in the sample solution (102), the display unit (304) to display that microbiota analysis is necessary for the sample solution (102).

15. The program according to claim 14,
wherein the program causes, in the system further including an analysis device capable of performing microbiota analysis, the diagnosing apparatus (803), the server (1001), and the analysis device being configured to communicate with each other via the network, the computer to further execute the procedures of:
receiving the specific response determined to be abnormal by comparing the numerical value and the reference, of the electrochemical response obtained by the diagnosing apparatus (803), and a sample ID corresponding to the sample solution (102);
receiving a result of a microbiota analysis of the sample solution (102) and the sample ID from the analysis device;
deciding presence or absence of the pathogenic microbes in the sample solution (102) from the result of the microbiota analysis; and
prompting, where it is decided from the result of the microbiota analysis that the pathogenic microbes have not been generated in the sample solution (102), the diagnosing apparatus (803) to change the reference.

## Patentansprüche

1. Diagnosevorrichtung (100; 300; 600; 700; 803), umfassend:
eine Zelle (101), die so konfiguriert ist, dass sie eine Probenlösung (102) einführt, die Körperflüssigkeit enthält, die von einem Probanden (804) entnommen wurde;
mindestens zwei Elektroden (103, 104; 501, 502), die in der Zelle (101) angeordnet sind und mit der Probenlösung (102) in Kontakt kommen;
eine Spannungsanlegeeinheit (105; 401), die so konfiguriert ist, dass sie eine Spannung zwischen den Elektroden (103, 104; 501, 502) anlegt;
eine Messeinheit (106; 402), die so konfiguriert ist, dass sie eine elektrochemische Reaktion misst, wenn die Spannung angelegt wird;
eine Speichereinheit (108; 403), die so konfiguriert ist, dass sie eine Referenz speichert, die eine Beziehung zwischen einem aus der elektrochemischen Reaktion erhaltenen numerischen Wert und mindestens einem von einer Art einer Krankheit und einem Fortschreiten der Krankheit definiert; und
eine Analyseausgabeeinheit (109; 404), die so konfiguriert ist, dass sie den von der Testperson (804) erhaltenen numerischen Wert mit der Referenz vergleicht und Informationen zur Bestimmung mindestens einer der folgenden Angaben bereitstellt: der Art der Krankheit, die sich bei der Testperson (804) entwickelt hat, und des Fortschreitungsgrades der Krankheit, die sich bei der Testperson (804) entwickelt hat,
**dadurch gekennzeichnet, dass** die elektrochemische Reaktion in dem Fall, in dem pathogene Mikroben in der Probenlösung (102) vorhanden sind, einen Stoffwechselstrom aufgrund der Oxidation organischer Substanzen umfasst.

2. Diagnosevorrichtung (100; 300; 600; 700; 803) gemäß Anspruch 1, wobei
der numerische Wert ein Absolutwert eines von der Messeinheit (106; 402) gemessenen Stroms oder eine Differenz zwischen der unter Verwendung von Körperflüssigkeit, die von der Person (804) gewonnen wurde, gemessenen elektrochemischen Reaktion und der unter Verwendung von Körperflüssigkeit, die von mindestens einer der Personen (804) in einem gesunden Zustand und einer gesunden Person, die sich von der Person (804) unterscheidet, gewonnen wurde, gemessenen elektrochemischen Reaktion ist.

3. Diagnosevorrichtung (100; 300; 600; 700; 803) gemäß Anspruch 1 oder 2, wobei die Körperflüssigkeit Speichel ist.

4. Diagnosevorrichtung (100; 300; 600; 700; 803) gemäß Anspruch 3, wobei die Krankheit mindestens eine von Karies und einer Parodontalerkrankung ist.

5. Diagnosevorrichtung (100; 300; 600; 700; 803) gemäß einem der Ansprüche 1 bis 4, wobei
die Speichereinheit (108; 403) ferner so konfiguriert ist, dass sie die gemessene elektrochemische Reaktion aufzeichnet, und
die Analyseausgabeeinheit (109; 404) ferner so konfiguriert ist, dass sie die durch Messung der Probenlösung (102) erhaltene elektrochemische Reaktion mit der elektrochemischen Reaktion vergleicht, die durch eine vorangegangene Messung vor der Messung erhalten und in der Speichereinheit (108; 403) aufgezeichnet wurde, und Informationen zur Bestimmung mindestens eines der folgenden Punkte auf der Grundlage des Vergleichsergebnisses bereitzustellen: die Art der Erkrankung, die sich bei dem Probanden (804) entwickelt hat, und den Fortschritt der Erkrankung, die sich bei dem Probanden (804) entwickelt hat.

6. Diagnosevorrichtung (100; 300; 600; 700; 803) gemäß einem der Ansprüche 1 bis 5, wobei
die Zelle (101) ferner so konfiguriert ist, dass sie hermetisch verschließbar ist.

7. Diagnosevorrichtung (300; 700; 803) gemäß einem der Ansprüche 1 bis 6, wobei der kürzeste Abstand zwischen den Elektroden (103, 104; 501, 502) in der Zelle (101) ein Abstand ist, bei dem eine der Zellen eines für die Krankheit verantwortlichen Mikroorganismus kurzgeschlossen werden kann.

8. Diagnosevorrichtung (300; 700; 803) gemäß Anspruch 7, wobei
der kürzeste Abstand zwischen den Elektroden (103, 104; 501, 502) in der Zelle (101) 700 nm bis 2.000 nm beträgt.

9. Diagnosevorrichtung (700; 803) gemäß einem der Ansprüche 1 bis 8, das ferner umfasst
eine Referenzerzeugungsvorrichtung (701), die so konfiguriert ist, dass sie die Referenz durch maschinelles Lernen auf der Grundlage der elektrochemischen Reaktion erzeugt.

10. Diagnosevorrichtung (803) gemäß einem der Ansprüche 1 bis 9, das ferner umfasst:
eine Übertragungseinheit, die so konfiguriert ist, dass sie die elektrochemische Reaktion über eine Kommunikationsleitung an einen externen Server überträgt; und
eine Empfangseinheit, die so konfiguriert ist, dass sie mindestens eine der folgenden Informationen empfängt: eine Konsultationsfrist und den erwarteten Krankheitsverlauf, die durch maschinelles Lernen in dem externen Server auf der Grundlage der von der Übertragungseinheit übertragenen elektrochemischen Reaktion berechnet wurden.

11. Analyseverfahren, umfassend:
einen Schritt des Einführens einer Probenlösung (102), die von einem Probanden (804) entnommene Körperflüssigkeit enthält, in eine Zelle (101), in der mindestens zwei Elektroden (103, 104; 501, 502) angeordnet sind, so dass sie mit den Elektroden (103, 104; 501, 502) in Kontakt zu bringen;
einen Schritt des Messens einer elektrochemischen Reaktion, wenn eine Spannung zwischen den Elektroden (103, 104; 501, 502) angelegt wird; und
einen Schritt des Vergleichens einer Referenz, die eine Beziehung zwischen einem aus der elektrochemischen Reaktion erhaltenen numerischen Wert und mindestens einem von einer Art einer Krankheit und einem Fortschreiten der Krankheit definiert, mit dem von der Person (804) erhaltenen numerischen Wert, um Informationen zum Bestimmen mindestens eines von der Art der Krankheit, die sich bei der Person (804) entwickelt hat, und dem Fortschreiten der Krankheit, die sich bei der Person (804) entwickelt hat, bereitzustellen,
**dadurch gekennzeichnet, dass** die elektrochemische Reaktion in dem Fall, dass pathogene Mikroben in der Probenlösung (102) vorhanden sind, einen Stoffwechselstrom aufgrund der Oxidation organischer Substanzen umfasst.

12. Analyseverfahren gemäß Anspruch 11, wobei
die Körperflüssigkeit Speichel ist.

13. Analyseverfahren gemäß Anspruch 12, wobei
die Krankheit mindestens eine von Karies und einer Parodontalerkrankung ist.

14. Programm, das in einem System, das eine Diagnosevorrichtung (803) mit den Merkmalen eines der Ansprüche 1 bis 10 und einen Server (1001), der ein Computer ist, umfasst, die Diagnosevorrichtung (803) und den Server (1001) so konfiguriert, dass sie über ein Netzwerk miteinander kommunizieren,
wobei die Diagnosevorrichtung (803) ferner umfasst
eine Anzeigeeinheit (304) umfasst,
wobei der Computer die folgenden Verfahren ausführt:
Empfangen einer spezifischen Reaktion, die durch Vergleichen des numerischen Werts und des Referenzwerts der von der Diagnosevorrichtung (803) erhaltenen elektrochemischen Reaktion als abnormal bestimmt wurde;
Anwenden der spezifischen Reaktion auf ein Schätzmodell, um die Bildung der pathogenen Mikroben in der Probenlösung (102) zu schätzen, wobei das Schätzmodell darauf trainiert ist, bei Eingabe einer elektrochemischen Reaktion das Vorhandensein oder Nichtvorhandensein der für die Erkrankung verantwortlichen pathogenen Mikroben zu schätzen, wobei das Training im Voraus im Schätzmodell durchgeführt wurde, indem Daten trainiert wurden, die eine elektrochemische Reaktion einer Trainingsprobenlösung, die sich von der Probenlösung unterscheidet, und ein Mikrobiota-Analyseergebnis der Trainingsprobenlösung (102) umfassen; und
Auffordern der Anzeigeeinheit (304), anzuzeigen, dass eine Mikrobiota-Analyse für die Probenlösung (102) erforderlich ist, wenn die Bildung der pathogenen Mikroben in der Probenlösung (102) zu erwarten ist.

15. Programm gemäß Anspruch 14,
wobei das Programm in dem System, das ferner eine Analysevorrichtung umfasst, die eine Mikrobiota-Analyse durchführen kann, wobei die Diagnosevorrichtung (803), der Server (1001) und die Analysevorrichtung so konfiguriert sind, dass sie über das Netzwerk miteinander kommunizieren, den Computer veranlasst, ferner die folgenden Verfahren auszuführen:
Empfangen der spezifischen Reaktion, die durch Vergleichen des numerischen Werts und der Referenz als abnormal bestimmt wurde, der elektrochemischen Reaktion, die durch die Diagnosevorrichtung (803) erhalten wurde, und einer Proben-ID, die der Probenlösung (102) entspricht;
Empfangen eines Ergebnisses einer Mikrobiota-Analyse der Messprobenlösung (102) und der Proben-ID von der Analysiervorrichtung;
Entscheiden über das Vorhandensein oder Nichtvorhandensein der pathogenen Mikroben in der Probenlösung (102) anhand des Ergebnisses der Mikrobiota-Analyse; und
Auffordern der Diagnosevorrichtung (803), den Referenzwert zu ändern, wenn anhand des Ergebnisses der Mikrobiota-Analyse entschieden wird, dass die pathogenen Mikroben in der Probenlösung (102) nicht erzeugt wurden.

## Revendications

1. Dispositif de diagnostic (100 ; 300 ; 600 ; 700 ; 803), comprenant :
une cellule (101) configurée pour introduire une solution échantillon (102) contenant un fluide corporel prélevé sur un sujet (804) ;
au moins deux électrodes (103, 104 ; 501, 502) qui sont disposées dans la cellule (101) et entrent en contact avec la solution échantillon (102) ;
une unité d'application de tension (105 ; 401) configurée pour appliquer une tension entre les électrodes (103, 104 ; 501, 502) ;
une unité de mesure (106 ; 402) configurée pour mesurer une réponse électrochimique lorsque la tension est appliquée ;
une unité de stockage (108 ; 403) configurée pour stocker une référence qui définit une relation entre une valeur numérique obtenue à partir de la réponse électrochimique et au moins l'un parmi un type de maladie et un état d'évolution de la maladie ; et
une unité de sortie d'analyse (109 ; 404) configurée pour comparer la valeur numérique obtenue à partir du sujet (804) avec la référence, et fournir des informations pour déterminer au moins l'un parmi le type de maladie qui s'est développé chez le sujet (804) et l'état d'avancement de la maladie qui s'est développée chez le sujet (804),
**caractérisé en ce que** la réponse électrochimique comprend, dans le cas où des microbes pathogènes sont présents dans la solution échantillon (102), un courant métabolique dû à l'oxydation de substances organiques.

2. Dispositif de diagnostic (100 ; 300 ; 600 ; 700 ; 803), selon la revendication 1, dans lequel
la valeur numérique est une valeur absolue d'un courant mesuré par l'unité de mesure (106 ; 402) ou une différence entre la réponse électrochimique mesurée à l'aide du fluide corporel obtenu à partir du sujet (804) et la réponse électrochimique mesurée à l'aide du fluide corporel obtenu à partir d'au moins l'un parmi le sujet (804) dans un état sain et une personne saine différente du sujet (804).

3. Dispositif de diagnostic (100 ; 300 ; 600 ; 700 ; 803), selon la revendication 1 ou 2, dans lequel
le fluide corporel est la salive.

4. Dispositif de diagnostic (100 ; 300 ; 600 ; 700 ; 803), selon la revendication 3, dans lequel
la maladie est au moins l'une parmi une carie et une maladie parodontale.

5. Dispositif de diagnostic (100 ; 300 ; 600 ; 700 ; 803), selon l'une quelconque des revendications 1 à 4, dans lequel
l'unité de stockage (108 ; 403) est en outre configurée pour enregistrer la réponse électrochimique mesurée, et
l'unité de sortie d'analyse (109 ; 404) est en outre configurée pour comparer la réponse électrochimique obtenue par mesure de la solution échantillon (102) avec la réponse électrochimique qui a été obtenue par une mesure précédente avant la mesure et enregistrée dans l'unité de stockage (108 ; 403), et fournir des informations pour déterminer au moins l'un parmi le type de la maladie qui s'est développée chez le sujet (804) et l'état d'avancement de la maladie qui s'est développée chez le sujet (804) sur la base de la référence provenant d'un résultat de la comparaison.

6. Dispositif de diagnostic (100 ; 300 ; 600 ; 700 ; 803), selon l'une quelconque des revendications 1 à 5, dans lequel
la cellule (101) est en outre configurée pour être hermétiquement scellable.

7. Dispositif de diagnostic (300 ; 700 ; 803) selon l'une quelconque des revendications 1 à 6, dans lequel
la distance la plus courte entre les électrodes (103, 104 ; 501, 502) dans la cellule (101) est une distance à laquelle l'une des cellules d'un microbe responsable de la maladie peut être court-circuitée.

8. Dispositif de diagnostic (300 ; 700 ; 803) selon la revendication 7, dans lequel la distance la plus courte entre les électrodes (103, 104 ; 501, 502) dans la cellule (101) est comprise entre 700 nm et 2 000 nm.

9. Dispositif de diagnostic (700 ; 803) selon l'une quelconque des revendications 1 à 8, comprenant en outre
un dispositif de création de référence (701) configuré pour créer la référence par apprentissage automatique sur la base de la réponse électrochimique.

10. Dispositif de diagnostic (803) selon l'une quelconque des revendications 1 à 9, comprenant en outre :
une unité de transmission configurée pour transmettre la réponse électrochimique à un serveur externe via une ligne de communication ; et
une unité de réception configurée pour recevoir au moins l'une parmi une date limite de consultation et une évolution prévue de la maladie qui a été calculée par apprentissage automatique dans le serveur externe sur la base de la réponse électrochimique transmise depuis l'unité de transmission.

11. Procédé d'analyse, comprenant :
une étape consistant à introduire, dans une cellule (101) dans laquelle au moins deux électrodes (103, 104 ; 501, 502) sont disposées, une solution échantillon (102) contenant un fluide corporel prélevé sur un sujet (804) de manière à entrer en contact avec les électrodes (103, 104 ; 501, 502) ;
une étape consistant à mesurer une réponse électrochimique lorsqu'une tension est appliquée entre les électrodes (103, 104 ; 501, 502) ; et
une étape consistant à comparer une référence définissant une relation entre une valeur numérique obtenue à partir de la réponse électrochimique et au moins l'un parmi un type de maladie et un état d'évolution de la maladie avec la valeur numérique obtenue à partir du sujet (804) afin de fournir des informations permettant de déterminer au moins l'un parmi le type de maladie qui s'est développé chez le sujet (804) et l'état d'évolution de la maladie qui s'est développé chez le sujet (804),
**caractérisé en ce que** la réponse électrochimique comprend, dans le cas où des microbes pathogènes sont présents dans la solution échantillon (102), un courant métabolique dû à l'oxydation de substances organiques.

12. Procédé d'analyse selon la revendication 11, dans lequel
le fluide corporel est la salive.

13. Procédé d'analyse selon la revendication 12, dans lequel
la maladie est au moins l'une parmi les caries et une maladie parodontale.

14. Programme qui, dans un système comprenant un dispositif de diagnostic (803) présentant les caractéristiques de l'une quelconque des revendications 1 à 10 et un serveur (1001) qui est un ordinateur, le dispositif de diagnostic (803) et le serveur (1001) étant configurés pour communiquer entre eux via un réseau,
le dispositif de diagnostic (803) comprenant en outre
une unité d'affichage (304),
l'ordinateur pour exécuter les procédures suivantes :
recevoir une réponse spécifique déterminée comme anormale en comparant la valeur numérique et la référence de la réponse électrochimique obtenue par le dispositif de diagnostic (803) ;
appliquer la réponse spécifique à un modèle d'estimation pour estimer la génération des microbes pathogènes dans la solution échantillon (102), le modèle d'estimation étant appris à estimer, lorsqu'une réponse électrochimique est entrée, la présence ou l'absence de génération des microbes pathogènes responsables de la maladie, l'apprentissage ayant été effectué à l'avance dans le modèle d'estimation par des données d'apprentissage comprenant une réponse électrochimique d'une solution d'échantillon d'apprentissage différente de la solution d'échantillon et un résultat d'analyse du microbiote de la solution d'échantillon d'apprentissage (102) ; et
demander, lorsque la génération des microbes pathogènes est attendue dans la solution échantillon (102), à l'unité d'affichage (304) d'afficher qu'une analyse du microbiote est nécessaire pour la solution échantillon (102).

15. Programme selon la revendication 14,
dans lequel le programme amène, dans le système comprenant en outre un dispositif d'analyse capable d'effectuer une analyse du microbiote, le dispositif de diagnostic (803), le serveur (1001) et le dispositif d'analyse étant configurés pour communiquer entre eux via le réseau, l'ordinateur à exécuter en outre les procédures suivantes :
recevoir la réponse spécifique déterminée comme anormale en comparant la valeur numérique et la référence, de la réponse électrochimique obtenue par le dispositif de diagnostic (803), et un identifiant d'échantillon correspondant à la solution échantillon (102) ;
recevoir un résultat d'une analyse microbiologique de la solution échantillon de mesure (102) et l'identifiant d'échantillon provenant du dispositif d'analyse ;
décider de la présence ou de l'absence de microbes pathogènes dans la solution échantillon (102) à partir du résultat de l'analyse du microbiote ; et
demander, lorsqu'il est décidé à partir du résultat de l'analyse du microbiote que les microbes pathogènes n'ont pas été générés dans la solution échantillon (102), à le dispositif de diagnostic (803) de modifier la référence.
